# EUROPEAN PATENT APPLICATION

(11) **EP 2 093 191 A1**
(43) Date of publication of application: **26.08.2009**
(21) Application number: 07830713.9
(22) Date of filing: 23.10.2007
(51) Int. Cl.: C01B 31/12, H01G 9/058

(54) **ACTIVATED CARBON AND METHOD FOR PRODUCING THE SAME**

(30) Priority: 09.11.2006 JP 2006303810
(71) Applicant: Sumitomo Chemical Company, Limited, Tokyo 104-8260 (JP)
(72) Inventor: KURAKANE, Kosuke, Ibaraki-shi Osaka 5670826 (JP); MURAKAMI, Chikara, Ibaraki-shi Osaka 5670841 (JP)
(74) Representative: Duckworth, Timothy John
(86) International application number: PCT/JP2007/070979
(87) International publication number: WO 2008/056554

(57) **Abstract**

An activated carbon obtained by activating a carbide of a compound (1) with an alkali metal hydroxide, wherein the compound (1) is obtained, for example, by dehydration condensation of a resorcinol and an aldehyde using an acid catalyst.

## Description

### Field of the Invention

The present invention relates to an activated carbon and a process for producing the same.

### Background Art

Currently, electric energy-storage devices having a capacity for storing large electric energy are required in the fields such as midnight-power storage and auxiliary power supplies for power failure. In the field of rechargeable transportation vehicles such as electric vehicles and hybrid vehicles and in the field of portable electric terminals such as mobile personal computers, cellular phones and portable audio devices, electric energy-storage devices having large capacity for storing electric energy per unit volume to operate for long duration in spite of the small size thereof are also required.

Electric double-layer capacitors are composed of an electrode, a separator and an electrolytic solution, and are expected as electric energy-storage devices storing electric power in a boundary surface (electric double-layer) formed between an electrolyte and an electrode due to absorption of the electrolyte dissolved in an electrolytic solution to the electrode. The stored energy is defined by (1/2) · C · V² (wherein C is electrostatic capacity (F) and V is voltage). In order to store more energy, the electrostatic capacity of electric energy-storage devices is needed to be enhanced, and in order to being compact and store more energy, enhancement of electrostatic capacity per unit volume is required.

As an electrode in electric double-layer capacitors, activated carbons are used for general purpose, and specifically, an activated carbon having pores mainly composed of micro pores (pore diameter is 20 Å or less) obtained by carbonization and activation of a raw material such as palm shell and the like is used. For improving electrostatic capacity, electric double-layer capacitors using a new activated carbon as an electrode are required now.

Recently, patent document 1 suggests that an activated carbon mainly composed of meso pores obtained by polymerizing resorcinol and formaldehyde into linear form in the presence of a basic catalyst to generate an organic aerogel uniformly having meso pores (pore diameter is 20 Å or more) followed by carbonizing and activating can be used as an electrode of an electric double-layer capacitor having large electrostatic capacity per unit volume.

Meanwhile, non-patent document 1 reports that a polymer substance composed of resorcinol and an aldehyde compound other than formaldehyde has a cyclic structure and that this cyclic structure can be carbonized. However, it is not disclosed that the resultant carbide can be used as an electrode material, and there is no disclosure regarding an electric double-layer capacitor containing an electrode made of this carbide, and regarding its electrostatic capacity.
[Patent document 1] U.S. Patent No. 4873218
[Non-patent document 1] Kazuhisa Murata, Takashi Masuda, Hisashi Ueda, Chemistry Express, Vol. 5, No. 8, pp. 606-608 (1990)

### Disclosure of the Invention

The present inventors have investigated to clarify that an electric double-layer capacitor using the carbide obtained from a cyclic structure obtained from resorcinol and aldehyde compound as an electrode does not show sufficient electrostatic capacity.

The object of the present invention is to provide an activated carbon which has large electrostatic capacity and which can be used as an electrode of an electric double-layer capacitor, and a method of producing the same.

That is, the present invention provides the following [1] to [7].
[1]. An activated carbon obtained by activating a carbide of a compound represented by the formula (1) with an alkali metal hydroxide: 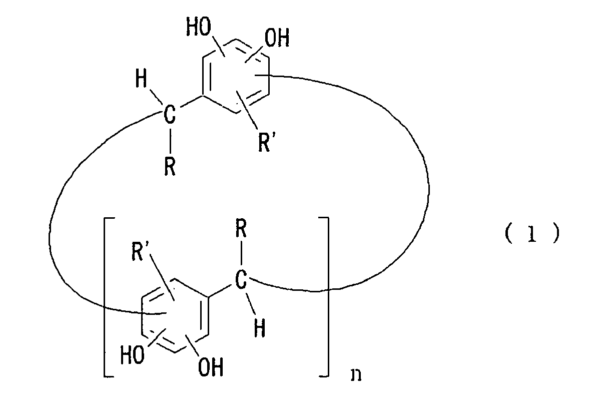 wherein, R represents a hydrocarbon group having 1 to 12 carbon atoms, and said hydrocarbon group may have a hydroxyl group, an alkyl group, an alkoxy group, an aryl group, an aryloxy group, a sulfonyl group, a halogen atom, a nitro group, a thioalkyl group, a cyano group, a carboxyl group, an amino group or an amide group, and R' represents a hydrogen atom or a methyl group, and n represents 3, 5 or 7.
[2]. The activated carbon according to [1], wherein R' in said formula (1) is a hydrogen atom.
[3]. The activated carbon according to [1] or [2], wherein the alkali metal hydroxide is potassium hydroxide, sodium hydroxide, or a mixture of potassium hydroxide and sodium hydroxide.
[4]. An electrode containing the activated carbon as described in any one of [1] to [3].
[5]. An electric double-layer capacitor having the electrode as described in [4].
[6]. A process for producing an activated carbon comprising carbonizing and activating a mixture of a compound represented by the formula (1) and an alkali metal hydroxide by heating under an inert gas atmosphere: 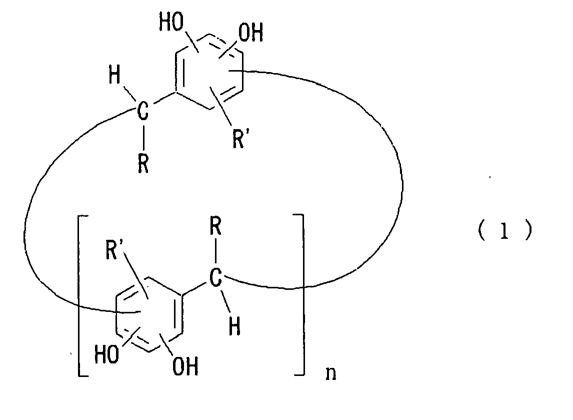 wherein, R represents a hydrocarbon group having 1 to 12 carbon atoms, and said hydrocarbon group may have a hydroxyl group, an alkyl group, an alkoxy group, an aryl group, an aryloxy group, a sulfonyl group, a halogen atom, a nitro group, a thioalkyl group, a cyano group, a carboxyl group, an amino group or an amide group, and R' represents a hydrogen atom or a methyl group, and n represents 3, 5 or 7.
[7]. A process for producing an activated carbon comprising carbonizing a compound represented by the formula (1) under an inert gas atmosphere, then, mixing an alkali metal hydroxide, and heating under an inert gas atmosphere to cause activation: 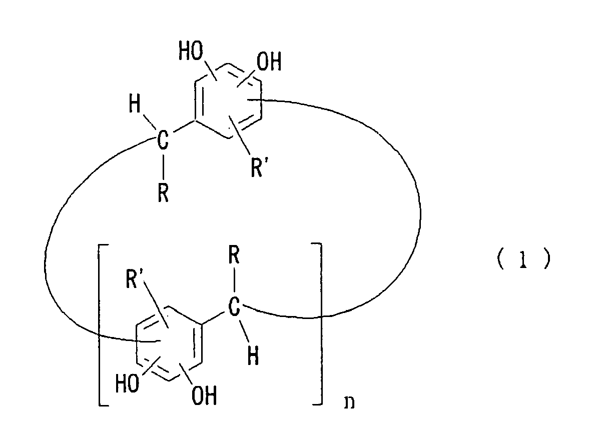 wherein, R represents a hydrocarbon group having 1 to 12 carbon atoms, and said hydrocarbon group may have a hydroxyl group, an alkyl group, an alkoxy group, an aryl group, an aryloxy group, a sulfonyl group, a halogen atom, a nitro group, a thioalkyl group, a cyano group, a carboxyl group, an amino group or an amide group, and R' represents a hydrogen atom or a methyl group, and n represents 3, 5 or 7.

### Brief Description of the Drawings

Fig. 1 is one embodiment of a coin-shaped electric double-layer capacitor (schematic diagram).
Fig. 2 is one embodiment of a wound electric double-layer capacitor (schematic diagram).
Fig. 3 is one embodiment of a laminated electric double-layer capacitor (schematic diagram).
Fig. 4 is another embodiment of a laminated electric double-layer capacitor different from the capacitor drawn in Fig. 3 (schematic diagram).
Fig. 5 is one embodiment of the laminated electric double-layer capacitor used in Examples and Comparative Examples of the present invention (schematic diagram).

### [Explanation of Numerals]

11: Metallic case
12: Current collector
13: Electrode
14: Separator
15: Metallic lid
16: Gasket
21: Metallic case
22: Current collector
23: Electrode
24: Separator
25: Electrode sealing pad
26: Lead
31: Metallic case
32: Current collector
33: Electrode
34: Separator
35: Lead
36: Terminal
37: Safety valve
41: Pressure plate and terminal
42: Current collector
43: Electrode
44: Separator
46: Gasket
51: Pressure plate
52: Current collector
53: Electrode
54: Separator
55: Insulating material

### Modes for Carrying Out the Invention

The present invention will be described in detail below.

In compounds represented by the formula (1), R represents a hydrocarbon group having 1 to 12 carbon atoms, and said hydrocarbon group may have a hydroxyl group, an alkyl group, an alkoxy group, an aryl group, an aryloxy group, a sulfonyl group, a halogen atom, a nitro group, a thioalkyl group, a cyano group, a carboxyl group, an amino group or an amide group.

Examples of R include alkyl groups such as a methyl group, an ethyl group and a butyl group, cycloalkyl groups such as a cyclohexyl group, and aromatic groups such as a phenyl group and a naphthyl group. Alternatively, examples of hydrocarbon groups having the above-mentioned substituent include aromatic groups to which an alkyl group is bonded such as a 2-tolyl group, a 3-tolyl group and a 4-tolyl group, and aromatic groups to which a hydroxyl group is bonded such as a 2-hydroxybenzyl group, a 3-hydroxybenzyl group and a 4-hydroxybenzyl group. Among them, aromatic groups to which the above-described substituent may be bonded are preferable, and aromatic groups to which a hydroxyl group or an alkyl group may be bonded are more preferable, from the standpoint of yield in carbonization.

R' in the formula (1) represents a hydrogen atom or a methyl group, and a hydrogen atom is preferable from the standpoint of easy production.

In the formula (1), n is 3, 5 or 7, and n is preferably 3 from the standpoint of easy production.

The hydroxyl group bonding to a benzene ring of the formula (1) together with R' is usually connected to an ortho-position and para-position of -CH(R)-.

The compound (1) has stereoisomers, and it may have a single configuration or may be a mixture of stereoisomers. If the compound (1) is produced using an acid catalyst as described later, a mixture of stereoisomers is usually obtained.

Specific examples of the compound (1) include compounds represented by the following formulae. Herein, R" includes groups shown in the right.

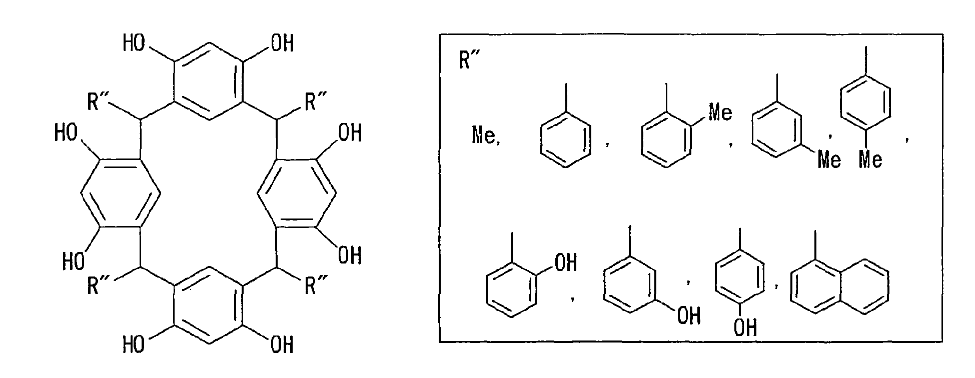

Examples of the process for producing the compound (1) include a method of subjecting a resorcinol optionally having a methyl group (hereinafter, occasionally referred to as resorcinols) and an aldehyde to dehydration condensation in the presence of an aqueous solvent with an acid catalyst as described in P.Timmerman et.al., Tetrahedron, 52, (1996) p2663-2704.

Examples of resorcinols used for producing the compound (1) include resorcinol, 2-methylresorcinol and 5-methylresorcinol. Resorcinol is preferable from the viewpoint of availability thereof.

Examples of the aldehydes used for producing the compound (1) include aliphatic aldehydes such as acetaldehyde, n-butylaldehyde, isobutylaldehyde, n-hexylaldehyde, n-dodecylaldehyde, 3-phenylpropionealdehyde and 5-hydroxypentanal; and
aromatic aldehydes such as benzaldehyde, 1-naphthaldehyde, 2-methylbenzaldehyde, 3-methylbenzaldehyde, 4-methylbenzaldehyde, 2-hydroxybenzaldehyde, 3-hydroxybenzaldehyde, 4-hydroxybenzaldehyde, 4-t-butylbenzaldehyde, 4-phenylbenzaldehyde, 2-methoxybenzaldehyde, 3-methoxybenzaldehyde, 4-methoxybenzaldehyde, 2-chlorobenzaldehyde, 3-chlorobenzaldehyde, 4-chlorobenzaldehyde, 2-bromobenzaldehyde, 3-bromobenzaldehyde, 4-bromobenzaldehyde, 2-fluorobenzaldehyde, 3-fluorobenzaldehyde, 4-fluorobenzaldehyde, 2-methylthiobenzaldehyde, 3-methylthiobenzaldehyde, 4-methylthiobenzaldehyde, 2-carboxybenzaldehyde, 3-carboxybenzaldehyde, 4-carboxybenzaldehyde, 3-nitrobenzaldehyde, 4-aminobenzaldehyde, 4-acetylaminobenzaldehyde and 4-cyanobenzaldehyde.

The used amount of aldehyde is usually about 1 to 3 moles and preferably about 1.2 to 2.5 moles per 1 mole of resorcinols.

Examples of the acid catalyst used for producing the compound (1) include hydrochloric acid, sulfuric acid, phosphoric acid, nitric acid and acetic acid. Among them, hydrochloric acid and sulfuric acid are preferable. The used amount of acid catalyst is usually about 0.001 to 3 moles per 1 mole of resorcinols.

The aqueous solvent used for producing the compound (1) is a mixture of water and an organic solvent mixable with water in optional ratios, or water. Specific examples thereof include alcohol solvents such as methanol, ethanol and i-propylalcohol and ether solvents such as tetrahydrofuran. When these solvents are used, they can be used alone or two or more kinds thereof can be mixed to be used.

As the aqueous solvent, an alcohol solvent having 3 or less carbon atoms or a mixture of water and an alcohol solvent having 3 or less carbon atoms is preferable, and an alcohol solvent having 3 or less carbon atoms is more preferable.

The ratio of used amount of the aqueous solvent and resorcinols is usually 0.5 to 5 parts by weight and preferably 1 to 2 parts by weight per 1 part by weight of the aqueous solvent.

Examples of processes for producing the compound (1) include a method of mixing together resorcinols, an aldehyde, an acid catalyst and an aqueous solvent, followed by stirring usually at 0 to 100°C and preferably 30 to 90°C to precipitate a compound (1) and then to collect the precipitated by filtration; a method of mixing an aldehyde with a mixture of resorcinols, an acid catalyst and an aqueous solvent usually at 0 to 100°C, and preferably 30 to 90°C to precipitate a compound (1) and then to collect the precipitated by filtration; a method of mixing resorcinols with a mixture of an aldehyde, an acid catalyst, and an aqueous solvent usually at 0 to 100°C and preferably 30 to 90°C to precipitate a compound (1) and then to collect the precipitated by filtration; and a method of mixing an acid catalyst with a mixture of resorcinols, an aldehyde, and an aqueous solvent usually at 0 to 100°C and preferably 30 to 90°C to precipitate a compound (1) and then to collect the precipitated by filtration.

In these methods, a poor solvent such as water may be added before collecting the precipitated compound (1) by filtration.

The compound (1) collected by filtration is usually dried at 10°C to about 100°C by ventilation, under reduced pressure, or the like. Alternatively, drying may be conducted after the compound (1) collected by fitration is washed with a hydrophilic organic solvent to replace. Examples of the hydrophilic organic solvent include alcohols such as methyl alcohol, ethyl alcohol, n-propyl alcohol and t-butyl alcohol; aliphatic nitriles such as acetonitrile; aliphatic ketones such as acetone; aliphatic sulfoxides such as dimethylsulfoxide; and aliphatic carboxylic acids such as acetic acid.

The activated carbon of the present invention is activated with an alkali metal hydroxide. Examples of the alkali metal hydroxide include lithium hydroxide, sodium hydroxide and potassium hydroxide. Among them, sodium hydroxide and potassium hydroxide are preferable, and potassium hydroxide is more preferable owing to a tendency of increasing the specific surface area of activated carbon and a tendency of increasing the electrostatic capacity per unit volume.

The alkali metal hydroxide may be used in the form of solid, and from the viewpoint of dispersibility and handling, it is preferably used in the form of aqueous solution. When used in the form of aqueous solution, the concentration of the solid component of the alkali metal hydroxide is usually 10 to 20 wt%.

The alkali metal hydroxide (solid) is used in an amount of 0.5 to 5-fold by weight and preferably 1 to 2-fold by weight based on the compound (1) before carbonization. When the amount of the alkali metal hydroxide (solid) is 0.5-fold by weight or more based on the compound (1), there is a tendency of increasing the specific surface area of activated carbon and therefore, it is preferable, and when 5-fold by weight or less, there is a tendency of increasing the electrostatic capacity per unit volume, and therefore, it is preferable.

Examples of the process for producing an activated carbon of the present invention include (i) a method in which a mixture of a compound represented by the formula (1) and an alkali metal hydroxide is carbonized and activated by heating under an atmosphere of an inert gas such as a rare gas such as He, Ne, Ar, Kr and Xe, and nitrogen gas, and (ii) a method in which a compound represented by the formula (1) is carbonized by heating under the inert gas atmosphere, then, an alkali metal hydroxide is mixed and the mixture is activated by heating under an inert gas atmosphere.

Further detailed examples of the method (i) include a method in which an aqueous solution of an alkali metal hydroxide is mixed with the compound (1) obtained by drying, and moisture is evaporated usually in a vacuum drying machine controlled at about 60°C to 100°C for about 4 to 24 hours, then, the mixture is carbonized and activated by heating usually at 400°C to 1000°C and preferably at 450°C to 900°C under an inert gas atmosphere.

The heating time of carbonization and activation is usually about 1 minute to 10 hours and preferably about 30 minutes to 3 hours. When the heating time is 10 hours or less, carbonization and activation progress sufficiently.

Next, the activated product is cooled, then, washed with water, then, dried, thereby, an activated carbon of the present invention can be obtained.

For example, in the method (ii), the compound (1) obtained by drying is heated usually at 400°C to 1000°C and preferably at 450°C to 900°C under an inert gas atmosphere, to obtain a carbide.

Heating for obtaining a carbide may be advantageously carried out for about 30 minutes to 3 hours from a point of termination of reduction in weight, and the heating time is usually about 1 minute to 10 hours and preferably about 30 minutes to 3 hours.

Subsequently, an aqueous solution of an alkali metal hydroxide of which concentration had been controlled to about 10 to 30 wt% is mixed with the carbide, then, each aqueous solution is treated in a vacuum drying machine at 80°C for about 12 hours to evaporate moisture, then, the mixture is activated by heating usually at 400°C to 1000°C and preferably at 450°C to 900°C under an inert gas atmosphere.

Next, the activated product is cooled, then, washed with water, then, dried, thereby, an activated carbon of the present invention can be obtained.

While the product activated by the method (i), the method (ii) or the like may be washed only with water to give an activated carbon of the present invention, recommended is a method in which, usually, washing with a mineral acid of which concentration has been controlled to 0.05 to 1 mol/L is performed approximately once or twice, then, washing with ion exchanged water is repeated approximately 5 to 10-times until pH of the solution becomes around 7, to obtain an activated carbon of the present invention. If washing is performed using an acid, an alkali metal hydroxide can be removed with good efficiency and therefore, it is preferable. Examples of the acid to be used for washing include mineral acids such as hydrochloric acid, sulfuric acid, nitric acid and phosphoric acid, and hydrochloric acid is preferable.

In the method of producing an activated carbon of the present invention, activation may progress with an activating agent other than alkali metal hydroxides, such as alkali metal halides such as potassium chloride and sodium chloride; alkaline earth metal halides such as zinc chloride and aluminum chloride; alkaline earth metal hydroxides such as calcium hydroxide; and oxidizing gases such as air, oxygen, water vapor and carbon dioxide in addition to activation by alkali metal hydroxides, and when an activating agent other than alkali metal hydroxides and oxidizing gases is used, activation is preferably performed with this activating agent in an amount of usually 1 wt% or less and preferably 0.1 to 0.8 wt% or less based on the alkali metal hydroxide, from the standpoint of electrostatic capacity. When an oxidizing gas is used as an activating agent, it is preferable that the volume occupying a space to be activated is controlled to usually 80 vol% or less, preferably 25 vol% or less, and the residual space is occupied with an inert gas, and activation is performed in this space, for the same reason.

The total pore volume of an activated carbon of the present invention is usually less than 1.1 ml/g and preferably 0.6 to 1.0 ml/g. When the total pore volume is less than 1.1 ml/g, electrostatic capacity per unit volume is improved, and therefore, it is preferable.

For obtaining such total pore volume, the activation time and activation temperature may be appropriately set.

Herein, the total pore volume is calculated from nitrogen adsorption amount around a relative pressure of 0.95 in a nitrogen adsorption isothermal curve at liquid nitrogen temperature using AUTOSORB manufactured by YUASA IONICS.

Thus obtained activated carbon is used for electrodes of dry batteries, redox capacitors, hybrid capacitors, electric double-layer capacitors and the like, and for preservatives, deodorizers, adsorbing agents, catalysts, carriers, fillers, coloring agents, antistatic agents and the like. Among them, the activated carbon performs a remarkably excellent effect for electrodes.

When used as an electrode, the obtained activated carbon is ground to an average particle size of usually 50 µm or less, preferably 30 µm or less and more preferably 10 µm or less. By finely grinding the activated carbon, the bulk density of an electrode is improved, and the internal resistance can be decreased.

Herein, the average particle size means volume average particle size measured using a laser diffraction mode grain size distribution measurement apparatus SALD2000J (registered trademark, manufactured by Shimadzu Corp.) with an activated carbon dispersed in a neutral detergent-containing aqueous solution.

As the grinding method, suitable are methods of grinding using a grinding machine for fine grinding such as, for example, an impact wear grinder, centrifugal grinder, ball mill (tube mill, compound mill, conical ball mill, rod mill), vibration mill, colloid mill, friction disk mill, jet mill and the like.

As the grinding machine, a ball mill is usually used, and in this case, it is preferable that balls and grinding vessels are made of non-metals such as alumina, agate and the like for avoiding mixing of a metal powder.

The filling density of an activated carbon of the present invention is usually 0.5 to 0.8 g/ml, preferably 0.55 to 0.78 g/ml and more preferably 0.6 to 0.75 g/ml. Herein, the filling density in the present invention is a value measured according to a manual filling method described in JIS K1474 5. 7 . 1 (filling density of activated carbon) with a 5 ml measuring cylinder.

In the electrode containing an activated carbon of the present invention, a binder, a conductive agent and the like are used as raw materials so as to provide easy molding as an electrode.

As the process for producing an electrode, a mixture of an activated carbon, a binder, a conducting agent and the like is usually molded on a current collector. Specific examples of these methods include a method of coating a slurry mixture of an binder, a conducting agent, a solvent and the like on a current collector by doctor blade method or dipping the collector in the slurry mixture, followed by drying; a method of disposing a sheet, which is obtained by adding a solvent to an activated carbon, a binder, a conducting agent and the like, followed by mixing, molding and then drying, on a current collector with interposing a conductive adhesive and then subjecting to pressing and heating treatments and drying; and a method of molding a mixture of an activated carbon, a binder, a conducting agent, a liquid lubricant and the like on a current collector, removing the liquid lubricant to obtain a sheet and then streching the sheet in mono- or multi-axial directions.

When the electrode is formed in a sheet shape, the thickness thereof is about 50 to about 1000 µm.

Examples of materials for the current collector include metals such as nickel, aluminium, titanium, copper, gold, silver, platinum, aluminium alloy and stainless steel; materials formed by conducting plasma spraying or ark spraying of nickel, aluminum, zinc, copper, tin, lead or an alloy thereof to carbon material or activated carbon fibers; and conductive films wherein a conducting agent is dispersed in resins such as rubbers and styrene-ethylene-butylene-styrene copolymer (SEBS). Aluminum, which is lightweight, has superior electrical conductivity and is stable electrochemically, is particularly preferable.

Examples of the configurations of the current collector include foil, plate, mesh, net, lath, punching and emboss and a combination thereof (for example, meshed plate).

Bumpy surface may be formed on a surface of the current collector by etching processing.

Examples of the conducting agent include conductive carbons such as graphite, carbon black, acetylene black, Ketjenblack and activated carbons other than that of the invention; graphitic conductants such as natural graphites, thermally expandable graphites, flake graphites and expandable graphites; carbon fibers such as vapor-grown carbon fibers; fine powders or fibers of metals such as aluminum, nickel, copper, silver, gold and platinum; conductive metal oxides such as ruthenium oxide or titanium oxide; and conductive polymers such as polyaniline, polypyrrole, polythiophene, polyacetylene and polyacene.

Carbon black, acetylene black and Ketjenblack are particularly preferable in terms of enhancing conductivity effectively with small quantity thereof.

The blending amount of the conducting agent in the electrode is usually about 5 to about 50 parts by weight and preferably about 10 to about 30 parts by weight, based on 100 parts by weight of the activated carbon of the present invention.

Examples of the binder include polymers of fluorine compounds. Examples of the fluorine compounds include fluorinated alkyl (1 to 18 carbon atoms) (meth)acrylate, perfluoroalkyl (meth)acrylates [for example, perfluorododecyl (meth)acrylate, perfluoro-n-octyl (meth)acrylate and perfluoro-n-butyl (meth) acrylate], perfluoroalkyl-substituted alkyl (meth)acrylates [for example, perfluorohexylethyl (meth)acrylate and perfluorooctylethyl (meth)acrylate], perfluorooxyalkyl (meth)acrylates [for example, perfluorododecyloxyethyl (meth)acrylate and perfluorodecyloxyethyl (meth)acrylate and the like], fluorinated alkyl (1 to 18 carbon atoms) crotonate, fluorinated alkyl(1 to 18 carbon atoms) malate and fumarate, fluorinated alkyl(1 to 18 carbon atoms) itaconate, fluorinated alkyl-substituted olefines(having about 2 to 10 carbon atoms and about 1 to 17 fluorine atoms), perfluorohexylethylene, fluorinated olefin having about 2 to 10 carbon atoms and about 1 to 20 fluorine atoms whose fluorine atom binds to the double-bonded carbon atom, tetrafluoroethylene, trifluoroethylene, vinylidene fluoride and hexafluoropropylene.

Examples of the other binders include addition polymers of monomers having ethylenic double bond but not having fluorine atom. Examples of the monomer include (cyclo)alkyl(1 to 22 carbon atoms) (meth)acrylates [for example, methyl (meth)acrylate, ethyl (meth)acrylate, n-butyl (meth)acrylate, iso-butyl (meth)acrylate, cyclohexyl (meth)acrylate, 2-ethylhexyl (meth)acrylate, isodecyl (meth)acrylate, lauryl (meth)acrylate, octadecyl (meth)acrylate and the like]; (meth)acrylates containing an aromatic ring [for example, benzyl (meth)acrylate, phenylethyl (meth)acrylate and the like]; mono(meth)acrylates of alkylene glycol or dialkylene glycol(having 2 to 4 carbon atoms in its alkylene group) [for example, 2-hydroxyethyl (meth)acrylate, 2-hydroxypropyl (meth)acrylate, diethylene glycol mono(meth)acrylate]; (poly) glycerin (1 to 4 of polymerization degree) mono(meth)acrylate; (meth)acrylate-based monomers such as polyfunctional (meth)acrylates [for example, (poly)ethylene glycol(1 to 100 of polymerization degree) di(meth)acrylate, (poly)propylene glycol(1 to 100 of polymerization degree) di(meth)acrylate, 2,2-bis(4-hydroxyethylphenyl)propane di(meth)acrylate, trimethylolpropane tri(meth)acrylate and the like]; (meth)acrylamide-based monomers such as (meth)acrylamide and (meth)acrylamide derivatives [for example, N-methylol(meth)acrylamide, diacetone acrylamide and the like]; monomers containing a cyano group such as (meth)acrylonitrile, 2-cyanoethyl (meth)acrylate and 2-cyanoethyl acrylamide; styrene-based monomers such as styrene and styrene derivatives having 7 to 18 carbon atoms [foe example, α-methylstyrene, vinyltoluene, p-hydroxystyrene and divinylbenzene and the like]; diene-based monomers such as alkadiene having 4 to 12 carbon atoms [for example, butadiene, isoprene, chloroprene and the like]; alkenyl ester-based monomers such as vinyl ester of carboxylic acid(having 2 to 12 carbon atoms) [for example, vinyl acetate, vinyl propionate, vinyl butyrate, vinyl octanoate and the like], and (meth) allyl ester of carboxylic acid(having 2 to 12 carbon atoms) [for example, (meth)allyl acetate, (meth)allyl propionate, (meth)allyl octanoate and the like]; monomers containing an epoxy group such as glycidyl (meth)acrylate and (meth)allyl glycidyl ether; monoolefins such as monoolefin having 2 to 12 carbon atoms [for example, ethylene, propylene, 1-butene, 1-octene, 1-dodecene and the like]; monomers containing a chlorine atom, a bromine atom or an iodine atom, monomers containing halogen atoms excluding fluorine atom such as vinyl chloride and vinylidene chloride; (meth)acrylic acid such as acrylic acid and methacrylic acid; and monomers having conjugated double bonds such as butadiene and isoprene.

The addition polymers may be copolymers composed of a plurality of monomers such as ethylene-vinyl acetate copolymer, styrene-butadiene copolymer and ethylene-propylene copolymer. Alternatively, polymers of vinyl carboxylate may be partially or completely saponificated such as polyvinyl alcohol.

The binder may be copolymers composed of fluorine compounds and monomers having ethylenic double bond but not having fluorine atom.

Examples of other binder include polysaccharides and derivatives thereof such as starch, methylcellulose, carboxymethylcellulose, hydroxymethylcellulose, hydroxyethylcellulose, hydroxypropylcellulose, carboxymethylhydroxyethylcellulose and nitrocellulose; phenol resins; melamine resins; polyurethane resins; urea resins; polyimide resins; polyamide-imide resins; petroleum pitches and coal-tar pitches.

Among them, polymers of fluorine compound are preferable as the binder, and polytetrafluoroethylene which is a polymer of tetrafluoroethylene is particularly preferable.

As the binder, a plurality of binders may be used.

The blending amount of the binder in the electrode is usually about 0.5 to about 30 parts by weight and preferably about 2 to about 30 parts by weight, based on 100 parts by weight of activated carbon.

Examples of the solvent used for the binder include alcohols such as IPA (isopropyl alcohol), ethanol and methanol, ethers and ketones.

When the binder is thickened, plasticizers may be used in order to apply easily on a current collector.

The conducting adhesive is a mixture of the above-described conducting agent and the above-described binders. Among them, a mixture of carbon black and polyvinyl alcohol is preferable because of solvent free, easy preparation and enhanced preservability thereof.

The electrode is used for an electrode of, for example, dry batteries, redox capacitors, hybrid capacitors and electric double-layer capacitors. Among them, it is preferable for an electrode of electric double-layer capacitors because of having superior electrostatic capacity.

Herein, the redox capacitor is a device wherein an active material is included in an electrode to store electric power by oxidation-reduction reaction as described, for example, in the third chapter (from the 141 page) of "The leading edge of large capacity electric double-layer capacitors" compiled under the editorship of TAMURA HIDEO and published by NTS Inc, having a constitution wherein a similar separator used in the electric double-layer capacitor mentioned below is interposed between two electrodes and an electrolytic solution is filled therein. In the present invention, an electrolytic solution means a mixture of an electrolyte and a solvent.

Examples of the active material used for the redox capacitor include oxides of transition metals such as ruthenium, transition metal hydroxides and conductive polymers. The electrode includes the activated carbon of present the invention alone or a mixture of the activated carbon of the present invention and the conducting agent illustrated above of 1 to 60% by weight and the binder illustrated above of 2 to 30% by weight.

Examples of the electrolytic solution for the redox capacitor, when an oxide of transition metals such as ruthenium or transition metal hydroxide is used as the active material, include aqueous sulfuric acid solution, for example, under conditions described in JP 2002-359155 A. Alternatively, when using an organic acid as the electrolyte and an electrolytic solution dissolved in an organic solvent, for example, conditions described in JP 2002-267860 A are applied. When using a conductive polymer as the active material, an electrolyte capable of being dissolved in an organic solvent and dissociated may be applied, and examples thereof include lithium salts such as LiBF₄, LiPF₆ and LiClO₄. Especially, LiPF₆ is preferably used due to large ionization degree and favorable solubility thereof. Meanwhile, these electrolytes can be used alone or by combination of two or more kinds thereof. The concentration of the electrolyte in the electrolytic solution is preferably 0.5 to 1.5 mol/L in order to have favorable ionic conductivity. When the concentration of electrolyte is 0.5 mol/L or more, it is preferable to enhance the electrostatic capacity, and when 1.5 mol/L or less, it is preferable to enhance ionic conductivity due to reduction of viscosity of the electrolytic solution.

As the solvent contained in the electrolytic solution for the redox capacitor, an organic polar solvent illustrated for the electric double-layer capacitor mentioned below is preferably used. Among them, an aprotic organic solvent is preferably used, and for example, a solvent of cyclic carbonates, chain carbonates or cyclic esters or a mixture of at least two kinds thereof are preferably used. Examples of the cyclic carbonates include ethylene carbonate and propylene carbonate; examples of the chain carbonates include dimethyl carbonate, diethyl carbonate and methyl ethyl carbonate; and examples of the cyclic esters include γ-butyrolactone and γ-valerolactone. These may be used alone or two or more kinds thereof may be mixed to use. Meanwhile, the electrolytic solution is required to have high dielectric constant to assist dissociation of electrolyte as well as low viscosity not to disturb mobility of ions and further high resistance against electrochemical oxidation and reduction. Therefore, the carbonates are particularly preferable as the solvent, and, for example, it is preferable to mix ethylene carbonate or the like as a solvent having high dielectric constant with diethyl carbonate or the like as a solvent having low viscosity to use.

The hybrid capacitor is a device in which electric power is stored, during charging, by introducing lithium ions into layers of carbon such as graphite at the negative electrode and drawing anions of electrolyte on the electrode surface at the positive electrode, resulting of formation of electric double layer. This capacitor is constituted by using a similar electrode to a negative electorde of lithium-ion secondary battery as the negative electrode, using the above-mentioned electrode of the present invention as the positive electrode, interposing a similar separator to that of the electric double-layer capacitor mentioned below between the positive and negative electrodes and filling an electrolytic solution therein. Specifically, as the electrode of negative electrode, those described in the third section of the first chapter (from the 25 page) of "Lithium secondary batteries in the next generation" compiled under the editorship of TAMURA HIDEO, published by NTS Inc., can be used.

As the electrolyte for hybrid capacitor, combinations of inorganic anions and lithium cation are usually used, and a combination of lithium cation and at least one inorganic anion selected from the group consisting of BF₄⁻, PF₆⁻ and ClO₄⁻ is preferable.

As the organic polar solvent contained in the electrolytic solution for the hybrid capacitor, a solvent mainly composed of at least one kind of the group consisting of carbonates and lactones is usually used. Specifc examples thereof include cyclic carbonates such as propylene carbonate, ethylene carbonate and butylene carbonate, chain carbonates such as dimethyl carbonate, ethyl methyl carbonate and diethyl carbonate, and γ-butyrolactone, and preferable solvent is a mixed solvent of ethylene carbonate and one or more kinds of chain carbonates, γ-butyrolactone alone, a mixed solvent of γ-butyrolactone and one or more kinds of chain carbonates or the like.

Additives such as those illustrated in the section about electric double-layer capacitor may be used.

The electrode including the active carbon of the present invention is preferably used for an electrode of the electric double-layer capacitor because it has an excellent electrostatic capacity. The electric double-layer capacitor is explained in detail below.

The electric double-layer capacitor comprising an electrode including the active carbon of the present invention is a capacitor characterized by including the above-mentioned electrode. Specific examples thereof include a capacitor wherein a separator independently is disposed between the two electrodes of positive and negative electrodes, which are above-mentioned electrodes, and an electrolytic solution is filled between the two electrodes of positive and negative electrodes, which are above-mentioned electrodes, and a capacitor wherein a solid electrolyte (a gel electrolyte) is filled between the two electrodes as positive and negative electrodes, which are above-mentioned electrodes.

According to charging, the positive electrode is positively charged resulting formation of an electric double layer at the boundary of the positive electrode by negative electrolyte as well as the negative electrode is negatively charged resulting formation of an electric double layer at the boundary of the negative electrode by positive electrolyte, consequently electric energy is stored. When the charging is discontinued, the electric double layers are maintained, and when it is discharged, the electric double layers are disappeared to release an electric energy.

The electric double-layer capacitor may be a single cell including positive and negative electrodes, or may be one further combined of plurality cells.

The solid electrolyte is one wherein an electrolyte mentioned below is dispersed in a resin, and an organic polar solvent also mentioned below may be further dispersed therein. Specific examples thereof include gel electrolytes described at the 79 page of "The leading edge of large capacity electric double-layer capacitors" compiled under the editorship of TAMURA HIDEO, published by NTS Inc., and solid electrolytes disclosed in JP 2004-172346 A and cited documents therein, JP 2004-303567 A and cited documents therein, JP 2003-68580 A and cited documents therein and JP 2003-257240 A.

The electric double-layer capacitor including the active carbon of the present invention is preferably an electric double-layer capacitor wherein a separator is independently disposed between two electrodes as positive and negative electrodes, which are electrodes including the active carbon of the present invention, and an electrolytic solution is filled between the separator and electrodes, and therefore, this electric double-layer capacitor is explained in detail, hereinafter.

Examples of the configurations of the electric double-layer capacitor include coin-shaped, wound, laminated and accordion formations.

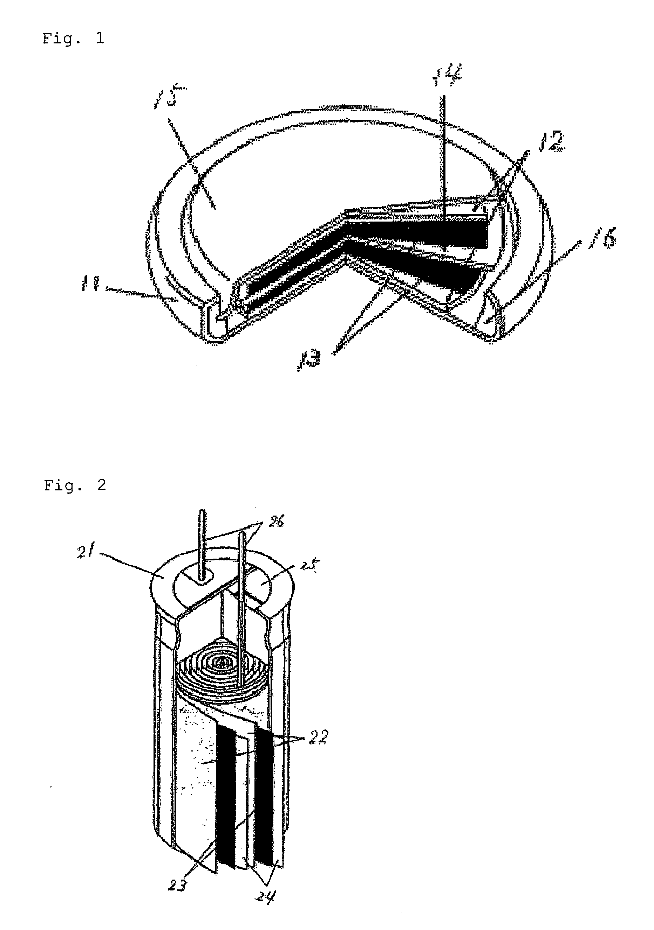

Examples of the method for producing a coin-shaped capacitor include a method comprising layering a current collector (12), an electrode (13), a separator (14), an electrode (13) and a current collector (12) in this order within a metallic case (11) made of stainless steel or the like, filling the case with an electrolytic solution and then sealing with a metallic lid (15) and gasket (16) is illustrated, as shown in Fig. 1.

Examples of the method for producing a wound capacitor include a method comprising applying a slurry mixture containing the above-mentioned activated carbon on a current collector (22) and then drying to prepare a laminated sheet composed of the current collector (22) and an electrode (23), winding two of the sheets with interposing a separator (24) between them and then housing this in a metallic case (21) made of aluminium, stainless steel or the like together with an electrode sealing pad (25), as shown in Fig. 2.

Meanwhile, the current collector is previously equipped with a lead, and electric energy is charged or discharged through a lead (26) provided to one layered sheet as a positive electrode and another lead (26) provided to another layered sheet as a negative electrode.

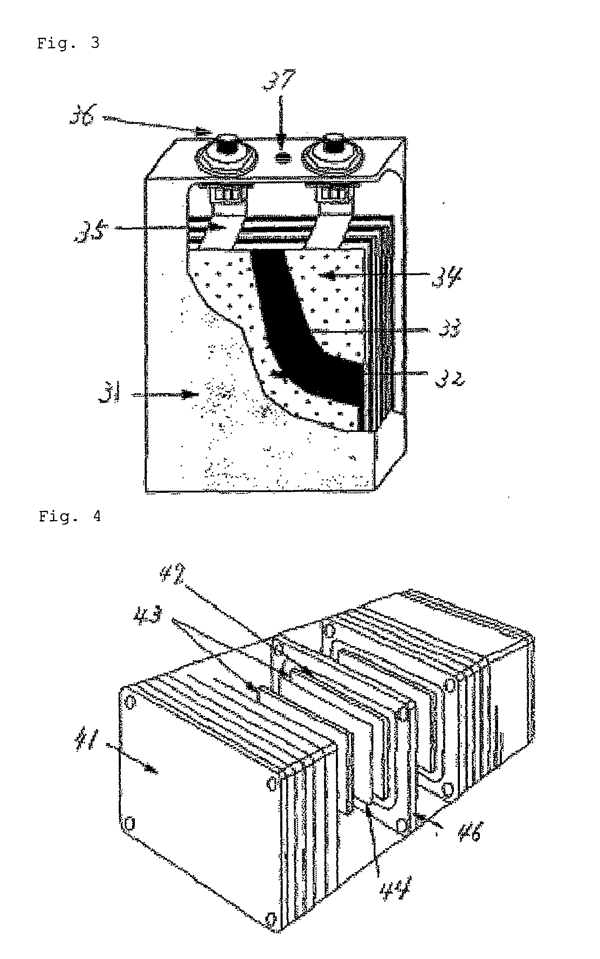

Examples of the method for producing a layered capacitor include a method comprising alternately laminating a layered sheet composed of a current collector (32) and an electrode (33) on a separator (34), housing this layers in a metallic case (31) made of aluminium, stainless steel or the like, filling the case with an electrolytic solution, alternately connecting the current collectors to a lead (35) and then sealing as shown in Fig. 3; a method comprising alternately connecting with pressure a layer sheet composed of a current collector (42) and an electrode (43) on a separator (44), sealing the outer layer with rubber materials or the like, filling an electrolytic solution therein and then sealing, as shown in Fig. 4. Alternatively, it may be formed as a bipolar structure appropriately including a gasket (46) which is a structure possible to optionally adjust an application voltage.

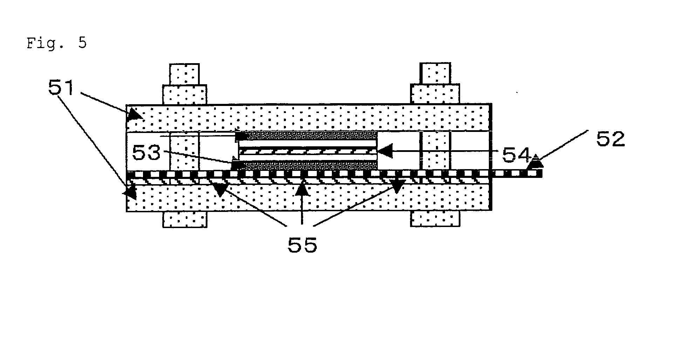

Examples of the present invention were carried out with an electric double-layer capacitor obtained by layering a sheet-shaped electrode (53), a separator (54), an electrode (53), an current collector (52) and insulating material (55) between pressure plates (51), filling an electrolytic solution between the separator (54) and the electrode (53), sealing the outer layer with a fluorine resin and then fastening by bolts as shown in Fig. 5. Meanwhile, the bolts are insulated from the current collector (52).

A method for producing an accordion capacitor is a method comprising layering two sheets of an electrode and a current collector in accordion-folding manner with interposing a separator between them and then preparing by the same way as applied to the layered capacitor.

The separator used for the electric double-layer capacitor functions to separate positive and negative electrodes and to retain an electrolytic solution, and a membrane having large ionic permeability, predetermined mechanical strength and electric insulating ability is used.

Examples of the separator include papers made of a viscose rayon, natural cellulose and the like, mixed papers made of fibers such as cellulose and polyester, electrolytic papers, kraft papers, manila papers, polyethylene non-woven fabrics, polypropylene non-woven fabrics, polyester non-woven fabrics, glass fibers, porous polyethylenes, porous polypropylenes, porous polyesters, aramid fibers, polybutyleneterephthalate non-woven fabrics, wholly aromatic p-polyamides, and fabrics or porous membranes of fuluorine-containing resins such as vinylidene fluoride, tetrafluoroethylene, copolymers of vinylidene fluoride and fluororubber.

The separator may be a molded article composed of powder particles of ceramics such as silica and the above-mentioned binders. The molded article is usually molded integrally with the positive and negative electrodes. Alternatively, a separator in which polyethylene or polypropylene is used may be mixed with surfactants or silica particles to enhance hydrophilicity thereof. Further, the separator may contain organic solvents such as acetone, plasticizer such as dibutylphthalate (DBP), and the like.

As the separator, a proton conductive polymer may be used.

As the separator, electrolytic papers, papers made of a viscose rayon or natural cellulose, kraft papers, manila papers, mixed papers made of cellulose or polyester fibers, polyethylene non-woven fabrics, polypropylene non-woven fabrics, polyester non-woven fabrics, sheets of Manila hemp and sheets of glass fibers are preferable.

The pore diameter of the separator is usually about 0.01 to about 10 µm. The thickness of the separator is usually about 1 to about 300 µm, and preferably about 5 to about 30 µm.

The separator may be a layered separator laminating separators having different pore ratios.

Electrolytes used for an electric double-layer capacitor are roughly classified into inorganic electrolytes and organic electrolytes, and examples of the inorganic electrolyte include acids such as sulfuric acid, hydrochloric acid and perchloric acid, bases such as sodium hydroxide, potassium hydroxide, lithium hydroxide and tetraalkylammonium hydroxide, and salts such as sodium chloride and sodium sulfate. As the inorganic electrolyte, an aqueous sulfuric acid solution is preferable because of excellent stability and low corrosive property against materials constituting an electric double-layer capacitor.

The concentration of the inorganic electrolyte is usually about 0.2 to about 5 mol (electrolyte) /L (electrolytic solution), and preferably about 1 to about 2 mol (electrolyte)/L (electrolytic solution). When the concentration is 0.2 to 5 mol/L, ion conductivity in the electrolytic solution can be secured.

The inorganic electrolyte is usually mixed with water to use in the form of electrolytic solution.

Examples of the organic electrolyte include combinations of inorganic anions such as BO₃³⁻ , F⁻, PF₆⁻, BF₄⁻ , AsF₆⁻ , SbF₆⁻, ClO₄⁻ , AlF₄⁻ , AlCl₄⁻, TaF₆⁻, NbF₆⁻, SiF₆²⁻, CN⁻, F(HF)ⁿ⁻ (in the formulae, n represents a numerical value of 1 or more and 4 or less) with organic cations described later, combinations of organic anions with organic cations described later, and combinations of organic anions with inorganic cations such as a lithium ion, sodium ion, potassium ion and hydrogen ion.

The organic cation is a cationic organic compound, and examples thereof include organic quaternary ammonium cations and organic quaternary phosphonium cations.

The organic quaternary ammonium cation is a quaternary ammonium cation in which a hydrocarbon group selected from the group consisting of alkyl groups (having 1 to 20 carbon atoms), cycloalkyl groups (having 6 to 20 carbon atoms), aryl groups (having 6 to 20 carbon atoms) and aralkyl groups (having 7 to 20 carbon atoms) is bonded to a nitrogen atom, and the organic quaternary phosphonium cation is a quaternary phosphonium cation in which the same hydrocarbon groups as described above are substituted to a phosphorus atom.

To the hydrocarbon group, a hydroxyl group, an amino group, a nitro group, a cyano group, a carboxyl group, an ether group, an aldehyde group or the like may be bonded.

Examples of main organic quaternary ammonium cations and organic quaternary phosphonium cations incude the followings.

### (Tetraalkylammonium cations)

tetramethylammonium, ethyltrimethylammonium, triethylmethylammonium, tetraethylammonium, tetra-n-propylammonium, tetra-n-butylammonium, diethyldimethylammonium, methyltri-n-propylammonium, tri-n-butylmethylammonium, ethyltri-n-butylammonium, tri-n-octylmethylammonium, ethyltri-n-octylammonium, diethylmethyl-i-propylammonium, diethylmethyl-n-propylammonium, ethyldimethyl-i-propylammonium, ethyldimethyl-n-propylammonium, diethylmethylmethoxyethylammonium, dimethylethylmethoxyethylammonium, benzyltrimethylammonium, (CF₃CH₂)(CH₃)₃N+, (CF₃CH₂)₂(CH₃)₂N+ and the like;

### (Ethylenediammonium cations)

N,N,N,N',N',N'-hexamethylethylenediammonium, N,N'-diethyl-N,N,N',N'-tetramethylethylenediammonium and the like;

### (bicyclic-ammonium cation represented by the following formula)

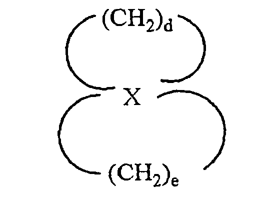

wherein X represents a nitrogen atom or a phosphorus atom, and d and e independently represent an integer of 4 to 6;

### (Guanidinium cations having an imidazolinium skeleton)

2-dimethylamino-1,3,4-trimethylimidazolinium, 2-diethylamino-1,3,4-trimethylimidazolinium, 2-diethylamino-1,3-dimethyl-4-ethylimidazolinium, 2-dimethylamino-1-methyl-3,4-diethylimidazolinium, 2-diethylamino-1-methyl-3,4-diethylimidazolinium, 2-diethylamino-1,3,4-triethylimidazolinium, 2-dimethylamino-1,3-dimethylimidazolinium, 2-diethylamino-1,3-dimethylimidazolinium, 2-dimethylamino-1-ethyl-3-methylimidazolinium, 2-diethylamino-1,3-diethylimidazolinium, 1,5,6,7-tetrahydro-1,2-dimethyl-12H-0imide[1,2a]imidazolini um, 1,5-dihydro-1,2-dimethyl-2H-imide[1,2a]imidazolinium, 1,5,6,7-tetrahydro-1,2-dimethyl-2H-pyrimido[1,2a]imidazolin ium, 1,5-dihydro-1,2-dimethyl-2H-pyrimido[1,2a]imidazolium, 2-dimethylamino-4-cyano-1,3-dimethylimidazolinium, 2-dimethylamino-3-cyanomethyl-1-methylimidazolinium, 2-dimethylamino-4-acetyl-1,3-dimethylimidazolinium, 2-dimethylamino-3-acetylmethyl-1-methylimidazolinium, 2-dimethylamino-4-methylcarbo-oxymethyl-1,3-dimethylimidazo linium, 2-dimethylamino-3-methylcarbo-oxymethyl-1-methylimidazolini um, 2-dimethylamino-4-methoxy-1,3-dimethylimidazolinium, 2-dimethylamino-3-methoxymethyl-1-methylimidazolinium, 2-dimethylamino-4-formyl-1,3-dimethylimidazolinium, 2-dimethylamino-3-formylmethyl-1-methylimidazolinium, 2-dimethylamino-3-hydroxyethyl-1-methylimidazolinium, 2-dimethylamino-4-hydroxymethyl-1,3-dimethylimidazolinium and the like;

### (Guanidinium cations having an imidazolium skeleton)

2-dimethylamino-1,3,4-trimethylimidazolium, 2-diethylamino-1,3,4-trimethylimidazolium, 2-diethylamino-1,3-dimethyl-4-ethylimidazolium, 2-dimethylamino-1-methyl-3,4-diethylimidazolium, 2-diethylamino-1-methyl-3,4-diethylimidazolium, 2-diethylamino-1,3,4-triethylimidazolium, 2-dimethylamino-1,3-dimethylimidazolium, 2-diethylamino-1,3-dimethylimidazolium, 2-dimethylamino-1-ethyl-3-methylimidazolium, 2-diethylamino-1,3-diethylimidazolium, 1,5,6,7-tetrahydro-1,2-dimethyl-2H-imide[1,2a]imidazolium, 1,5-dihydro-1,2-dimethyl-2H-imide[1,2a]imidazolium, 1,5,6,7-tetrahydro-1,2-dimethyl-2H-pyrimido[1,2a]imidazoliu m, 1,5-dihydro-1,2-dimethyl-2H-pyrimido[1,2a]imidazolium, 2-dimethylamino-4-cyano-1,3-dimethylimidazolium, 2-dimethylamino-3-cyanomethyl-1-methylimidazolium, 2-dimethylamino-4-acetyl-1,3-dimethylimidazolium, 2-dimethylamino-3-acetylmethyl-1-methylimidazolium, 2-dimethylamino-4-methylcarbo-oxymethyl-1,3-dimethylimidazo lium, 2-dimethylamino-3-methylcarbo-oxymethyl-1-methylimidazolium, 2-dimethylamino-4-methoxy-1,3-dimethylimidazolium, 2-dimethylamino-3-methoxymethyl-1-methylimidazolium, 2-dimethylamino-4-formyl-1,3-dimethylimidazolium, 2-dimethylamino-3-formylmethyl-1-methylimidazolium, 2-dimethylamino-3-hydroxyethyl-1-methylimidazolium, 2-dimethylamino-4-hydroxymethyl-1,3-dimethylimidazolium and the like;

### (Guanidinium cations having a tetrahydropyrimidinium skeleton)

2-dimethylamino-1,3,4-trimethyl-1,4,5,6-tetrahydropyrimidin ium, 2-diethylamino-1,3,4-trimethyl-1,4,5,6-tetrahydropyrimidini um, 2-diethylamino-1,3-dimethyl-4-ethyl-1,4,5,6-tetrahydropyrim idinium, 2-dimethylamino-1-methyl-3,4-diethyl-1,4,5,6-tetrahydropyri midinium, 2-diethylamino-1-methyl-3,4-diethyl-1,4,5,6-tetrahydropyrim idinium, 2-diethylamino-1,3,4-tetraethyl-1,4,5,6-tetrahydropyrimidin ium, 2-dimethylamino-1,3-dimethyl-1,4,5,6-tetrahydropyrimidinium, 2-diethylamino-1,3-dimethyl-1,4,5,6-tetrahydropyrimidinium, 2-dimethylamino-1-ethyl-3-methyl-1,4,5,6-tetrahydropyrimidi nium, 2-diethylamino-1,3-diethyl-1,4,5,6-tetrahydropyrimidinium, 1,3,4,6,7,8-hexahydro-1,2-dimethyl-2H-imide[1,2a]pyrimidini um, 1,3,4,6-tetrahydro-1,2-dimethyl-2H-imide[1,2a]pyrimidinium, 1,3,4,6,7,8-hexahydro-1,2-dimethyl-2H-pyrimido[1,2a]pyrimid inium, 1,3,4,6-tetrahydro-1,2-dimethyl-2H-pyrimido[1,2a]pyrimidini um, 2-dimethylamino-4-cyano-1,3-dimethyl-1,4,5,6-tetrahydropyri midinium, 2-dimethylamino-3-cyanomethyl-1-methyl-1,4,5,6-tetrahydropy rimidinium, 2-dimethylamino-4-acetyl-1,3-dimethyl-1,4,5,6-tetrahydropyr imidinium, 2-dimethylamino-3-acetylmethyl-1-methyl-1,4,5,6-tetrahydrop yrimidinium, 2-dimethylamino-4-methylcarbo-oxymethyl-1,3-dimethyl-1,4,5, 6-tetrahydropyrimidinium, 2-dimethylamino-3-methylcarbo-oxymethyl-1-methyl-1,4,5,6-te trahydropyrimidinium, 2-dimethylamino-4-methoxy-1,3-dimethyl-1,4,5,6-tetrahydropy rimidinium, 2-dimethylamino-3-methoxymethyl-1-methyl-1,4,5,6-tetrahydro pyrimidinium, 2-dimethylamino-4-formyl-1,3-dimethyl-1,4,5,6-tetrahydropyr imidinium, 2-dimethylamino-3-formylmethyl-1-methyl-1,4,5,6-tetrahydrop yrimidinium, 2-dimethylamino-3-hydroxyethyl-1-methyl-1,4,5,6-tetrahydrop yrimidinium, 2-dimethylamino-4-hydroxymethyl-1,3-dimethyl-1,4,5,6-tetrah ydro pyrimidinium and the like;

### (Guanidinium cations having a dihydropyrimidinium skeleton)

2-dimethylamino-1,3,4-trimethyl-1,4(6)-dihydropyrimidinium, 2-diethylamino-1,3,4-trimethyl-1,4(6)-dihydropyrimidinium, 2-diethylamino-1,3-dimethyl-4-ethyl-1,4(6)-dihydropyrimidin ium, 2-dimethylamino-1-methyl-3,4-diethyl-1,4(6)-dihydropyrimidi nium, 2-diethylamino-1-methyl-3,4-diethyl-1,4(6)-dihydropyrimidin ium, 2-diethylamino-1,3,4-tetraethyl-1,4(6)-dihydropyrimidinium, 2-dimethylamino-1,3-dimethyl-1,4(6)-dihydropyrimidinium, 2-diethylamino-1,3-dimethyl-1,4(6)-dihydropyrimidinium, 2-dimethylamino-1-ethyl-3-methyl-1,4(6)-dihydropyrimidinium, 2-diethylamino-1,3-diethyl-1,4(6)-dihydropyrimidinium, 1,6,7,8-tetrahydro-1,2-dimethyl-2H-imide[1,2a]pyrimidinium, 1,6-dihydro-1,2-dimethyl-2H-imide[1,2a]pyrimidinium, 1,6,7,8-tetrahydro-1,2-dimethyl-2H-pyrimido[1,2a]pyrimidini um, 1,6-dihydro-1,2-dimethyl-2H-pyrimido[1,2a]pyrimidinium, 2-dimethylamino-4-cyano-1,3-dimethyl-1,4(6)-dihydropyrimidi nium, 2-dimethylamino-3-cyanomethyl-1-methyl-1,4(6)-dihydropyrimi dinium, 2-dimethylamino-4-acetyl-1,3-dimethyl-1,4(6)-dihydropyrimid inium, 2-dimethylamino-3-acetylmethyl-1-methyl-1,4(6)-dihydropyrim idinium, 2-dimethylamino-4-methylcarbo-oxymethyl-1,3-dimethyl-1,4(6) -dihydropyrimidinium, 2-dimethylamino-3-methylcarbo-oxymethyl-1-methyl-1,4(6)-dih ydropyrimidinium, 2-dimethylamino-4-methoxy-1,3-dimethyl-1,4(6)-dihydropyrimi dinium, 2-dimethylamino-3-methoxymethyl-1-methyl-1,4(6)-dihydropyri midinium, 2-dimethylamino-4-formyl-1,3-dimethyl-1,4,5,6-tetrahydropyr imidinium, 2-dimethylamino-3-formylmethyl-1-methyl-1,4,5,6-tetrahydrop yrimidinium, 2-dimethylamino-3-hydroxyethyl-1-methyl-1,4,5,6-tetrahydrop yrimidinium, 2-dimethylamino-4-hydroxymethyl-1,3-dimethyl-1,4(6)-dihydro pyrimidinium and the like;

### (Pyrrolidinium cations)

N,N-dimethylpyrrolidinium, N-ethyl-N-methylpyrrolidinium, N-n-propyl-N-methylpyrrolidinium, N-n-butyl-N-methylpyrrolidinium, N,N-diethylpyrrolidinium and the like;

### (Piperidinium cations)

N,N-dimethylpiperidinium, N-ethyl-N-methylpiperidinium, N,N-diethylpiperidinium, N-n-propyl-N-methylpiperidinium, N-n-butyl-N-methylpiperidinium, N-ethyl-N-n-butylpiperidinium and the like;

### (Hexamethyleneiminium cations)

N,N-dimethylhexamethyleneiminium, N-ethyl-N-methylhexamethyleneiminium, N,N-diethylhexamethyleneiminium and the like;

### (Morpholinium cations)

N,N-dimethylmorpholinium, N-ethyl-N-methylmorpholinium, N-butyl-N-methylmorpholinium, N-ethyl-N-butylmorpholinium and the like;

### (Piperazinium cations)

N,N,N',N'-tetramethyl piperazinium, N-ethyl-N,N',N'-trimethyl piperazinium, N,N'-diethyl-N,N'-dimethylpiperazinium, N,N,N'-triethyl-N'-methyl piperazinium, N,N,N',N'-tetraethylpiperazinium and the like;

### (Tetrahydropyrimidinium cations)

1,3-dimethyl-1,4,5,6-tetrahydropyrimidinium, 1,2,3-trimethyl-1,4,5,6-tetrahydropyrimidinium, 1,3,4-trimethyl-1,4,5,6-tetrahydropyrimidinium, 1,3,5-trimethyl-1,4,5,6-tetrahydropyrimidinium, 1-ethyl-2,3-dimethyl-1,4,5,6-tetrahydropyrimidinium, 1-ethyl-3,4-dimethyl-1,4,5,6-tetrahydropyrimidinium, 1-ethyl-3,5-dimethyl-1,4,5,6-tetrahydropyrimidinium, 1-ethyl-3,6-dimethyl-1,4,5,6-tetrahydropyrimidinium, 2-ethyl-1,3-dimethyl-1,4,5,6-tetrahydropyrimidinium, 4-ethyl-1,3-dimethyl-1,4,5,6-tetrahydropyrimidinium, 5-ethyl-1,3-dimethyl-1,4,5,6-tetrahydropyrimidinium, 1,2,3,4-tetramethyl-1,4,5,6-tetrahydropyrimidinium, 1,2,3,5-tetramethyl-1,4,5,6-tetrahydropyrimidinium, 8-methyl-1,8-diazabicyclo[5.4.0]-7-undecenium, 5-methyl-1,5-diazabicyclo[4.3.0]-5-nonenium, 8-ethyl-1,8-diazabicyclo[5.4.0]-7-undecenium, 5-ethyl-1,5-diazabicyclo[4.3.0]-5-nonenium, 5-methyl-1,5-diazabicyclo[5.4.0]-5-undecenium, 5-ethyl-1,5-diazabicyclo[5.4.0]-5-undecenium, 1,2,3,4-tetramethyl-1,4,5,6-tetrahydropyrimidinium, 1,2,3,5-tetramethyl-1,4,5,6-tetrahydropyrimidinium, 1-ethyl-2,3,4-trimethyl-1,4,5,6-tetrahydropyrimidinium, 1-ethyl-2,3,5-trimethyl-1,4,5,6-tetrahydropyrimidinium, 1-ethyl-2,3,6-trimethyl-1,4,5,6-tetrahydropyrimidinium, 2-ethyl-1,3,4-trimethyl-1,4,5,6-tetrahydropyrimidinium, 2-ethyl-1,3,5-trimethyl-1,4,5,6-tetrahydropyrimidinium, 4-ethyl-1,2,3-trimethyl-1,4,5,6-tetrahydropyrimidinium, 4-ethyl-1,3,5-trimethyl-1,4,5,6-tetrahydropyrimidinium, 4-ethyl-1,3,6-trimethyl-1,4,5,6-tetrahydropyrimidinium, 5-ethyl-1,2,3-trimethyl-1,4,5,6-tetrahydropyrimidinium, 5-ethyl-1,3,4-trimethyl-1,4,5,6-tetrahydropyrimidinium, 1,2-diethyl-3,4-dimethyl-1,4,5,6-tetrahydropyrimidinium, 1,2-diethyl-3,5-dimethyl-1,4,5,6-tetrahydropyrimidinium, 1,2-diethyl-3,6-dimethyl-1,4,5,6-tetrahydropyrimidinium, 1,3-diethyl-2,4-dimethyl-1,4,5,6-tetrahydropyrimidinium, 1,3-diethyl-2,5-dimethyl-1,4,5,6-tetrahydropyrimidinium, 1,4-diethyl-2,3-dimethyl-1,4,5,6-tetrahydropyrimidinium, 1,4-diethyl-3,5-dimethyl-1,4,5,6-tetrahydropyrimidinium, 1,4-diethyl-3,6-dimethyl-1,4,5,6-tetrahydropyrimidinium, 1,5-diethyl-2,3-dimethyl-1,4,5,6-tetrahydropyrimidinium, 1,5-diethyl-3,4-dimethyl-1,4,5,6-tetrahydropyrimidinium, 1,5-diethyl-3,6-dimethyl-1,4,5,6-tetrahydropyrimidinium, 2,4-diethyl-1,3-dimethyl-1,4,5,6-tetrahydropyrimidinium, 2,5-diethyl-1,3-dimethyl-1,4,5,6-tetrahydropyrimidinium, 4,5-diethyl-1,3-dimethyl-1,4,5,6-tetrahydropyrimidinium, 4,6-diethyl-1,3-dimethyl-1,4,5,6-tetrahydropyrimidinium, 1,2,3,4,5-pentamethyl-1,4,5,6-tetrahydropyrimidinium, 1,2,3,4,6-pentamethyl-1,4,5,6-tetrahydropyrimidinium, 1,2,3,4,5,6-hexamethyl-1,4,5,6-tetrahydropyrimidinium, 4-cyano-1,2,3-trimethyl-1,4,5,6-tetrahydropyrimidinium, 3-cyanomethyl-1,2-dimethyl-1,4,5,6-tetrahydropyrimidinium, 2-cyanomethyl-1,3-dimethyl-1,4,5,6-tetrahydropyrimidinium, 4-acetyl-1,2,3-trimethyl-1,4,5,6-tetrahydropyrimidinium, 3-acetylmethyl-1,2-dimethyl-1,4,5,6-tetrahydropyrimidinium, 4-methylcarbo-oxymethyl-1,2,3-trimethyl-1,4,5,6-tetrahydrop yrimidinium, 3-methylcarbo-oxymethyl-1,2-dimethyl-1,4,5,6-tetrahydropyri midinium, 4-methoxy-1,2,3-trimethyl-1,4,5,6-tetrahydropyrimidinium, 3-methoxymethyl-1,2-dimethyl-1,4,5,6-tetrahydropyrimidinium, 4-formyl-1,2,3-trimethyl-1,4,5,6-tetrahydropyrimidinium, 3-formylmethyl-1,2-dimethyl-1,4,5,6-tetrahydropyrimidinium, 3-hydroxyethyl-1,2-dimethyl-1,4,5,6-tetrahydropyrimidinium, 4-hydroxymethyl-1,2,3-trimethyl-1,4,5,6-tetrahydropyrimidin ium, 2-hydroxyethyl-1,3-dimethyl-1,4,5,6-tetrahydropyrimidinium and the like;

### (Dihydropyrimidinium cations)

1,3-dimethyl-1,4- or -1,6-dihydropyrimidinium [which are collectively expressed with "1,3-dimethyl-1,4(6)-dihydropyrimidinium", and hereinafter same expression is used], 1,2,3-trimethyl-1,4(6)-dihydropyrimidinium, 1,2,3,4-tetramethyl-1,4(6)-dihydropyrimidinium, 1,2,3,5-tetramethyl-1,4(6)-dihydropyrimidinium, 8-methyl-1,8-diazabicyclo[5,4,0]-7,9(10)-undecadienium, 5-methyl-1,5-diazabicyclo[4,3,0]-5,7(8)-nonadienium, 4-cyano-1,2,3-trimethyl-1,4(6)-dihydropyrimidinium, 3-cyanomethyl-1,2-dimethyl-1,4(6)-dihydropyrimidinium, 2-cyanomethyl-1,3-dimethyl-1,4(6)-dihydropyrimidinium, 4-acetyl-1,2,3-trimethyl-1,4(6)-dihydropyrimidinium, 3-acetylmethyl-1,2-dimethyl-1,4(6)-dihydropyrimidinium, 4-methylcarbo-oxymethyl-1,2,3-trimethyl-1,4(6)-dihydropyrim idinium, 3-methylcarbo-oxymethyl-1,2-dimethyl-1,4(6)-dihydropyrimidi nium, 4-methoxy-1,2,3-trimethyl-1,4(6)-dihydropyrimidinium, 3-methoxymethyl-1,2-dimethyl-1,4(6)-dihydropyrimidinium, 4-formyl-1,2,3-trimethyl-1,4(6)-dihydropyrimidinium, 3-formylmethyl-1,2-dimethyl-1,4(6)-dihydropyrimidinium, 3-hydroxyethyl-1,2-dimethyl-1,4(6)-dihydropyrimidinium, 4-hydroxymethyl-1,2,3-trimethyl-1,4(6)-dihydropyrimidinium, 2-hydroxyethyl-1,3-dimethyl-1,4(6)-hydropyrimidinium and cations in which a hydrogen atom at 2-position of the dihydropyrimidinium cations described above is replaced with a fluorine atom and the like; 1,3,4,6,7,8-hexahydro-1,2-dimethyl-2H-pyrimido[1,2a]pyrimid inium;

### (Pyridinium cations)

N-methylpyridinium, N-ethylpyridinium, N-n-propylpyridinium, N-n-butylpyridinium, N-methyl-4-methylpyridinium, N-ethyl-4-methylpyridinium, N-n-propyl-4-methylpyridinium, N-n-butyl-4-methylpyridinium, N-methyl-3-methylpyridinium, N-ethyl-3-methylpyridinium, N-n-propyl-3-methylpyridinium, N-n-butyl-3-methylpyridinium, N-methyl-2-methylpyridinium, N-ethyl-2-methylpyridinium, N-n-propyl-2-methylpyridinium, N-n-butyl-2-methylpyridinium, N-methyl-2,4-dimethylpyridinium, N-ethyl-2,4-dimethylpyridinium, N-n-propyl-2,4-dimethylpyridinium, N-n-butyl-2,4-dimethylpyridinium, N-methyl-3,5-dimethylpyridinium, N-ethyl-3,5-dimethylpyridinium, N-n-propyl-3,5-dimethylpyridinium, N-n-butyl-3,5-dimethylpyridinium, N-methyl-4-dimethylaminopyridinium, N-ethyl-4-dimethylaminopyridinium, N-n-propyl-4-dimethylaminopyridinium, N-n-butyl-4-dimethylaminopyridinium and the like;

### (Picolinium cation)

N-methylpicolinium, N-ethylpicolinium and the like; (Imidazolinium-based cations) 1,2,3-trimethylimidazolinium, 1,2,3,4-tetramethylimidazolinium, 1,3,4-trimethyl-2-ethylimidazolinium, 1,3-dimethyl-2,4-diethylimidazolinium, 1,2-dimethyl-3,4-diethylimidazolinium, 1-methyl-2,3,4-triethylimidazolinium, 1,2,3,4-tetraethylimidazolinium, 1,3-dimethyl-2-ethylimidazolinium, 1-ethyl-2,3-dimethylimidazolinium, 1,2,3-triethylimidazolinium, 1,1-dimethyl-2-heptylimidazolinium, 1,1-dimethyl-2-(2'-heptyl)imidazolinium, 1,1-dimethyl-2-(3'-heptyl)imidazolinium, 1,1-dimethyl-2-(4'-heptyl)imidazolinium, 1,1-dimethyl-2-dodecylimidazolinium, 1,1-dimethylimidazolinium, 1,1,2-trimethylimidazolinium, 1,1,2,4-tetramethylimidazolinium, 1,1,2,5-tetramethylimidazolinium, 1,1,2,4,5-pentamethylimidazolinium, 1,2,3-trimethylimidazolinium, 1,3,4-trimethylimidazolinium, 1,2,3,4-tetramethylimidazolinium, 1,2,3,4-tetraethylimidazolinium, 1,2,3,5-pentamethylimidazolinium, 1,3-dimethyl-2-ethylimidazolinium, 1-ethyl-2,3-dimethylimidazolinium, 1-ethyl-3,4-dimethylimidazolinium, 1-ethyl-3,5-dimethylimidazolinium, 4-ethyl-1,3-dimethylimidazolinium, 1,2-diethyl-3-methylimidazolinium, 1,4-diethyl-3-methylimidazolinium, 1,5-diethyl-3-methylimidazolinium, 1,3-diethyl-2-methylimidazolinium, 1,3-diethyl-4-methylimidazolinium, 1,2,3-triethylimidazolinium, 1-ethyl-2,3,4-trimethylimidazolinium, 1-ethyl-2,3,5-trimethylimidazolinium, 1-ethyl-3,4,5-trimethylimidazolinium, 2-ethyl-1,3,4-trimethylimidazolinium, 4-ethyl-1,2,3-trimethylimidazolinium, 1,2-diethyl-3,4-dimethylimidazolinium, 1,3-diethyl-2,4-dimethylimidazolinium, 1,4-diethyl-2,3-dimethylimidazolinium, 2,4-diethyl-1,3-dimethylimidazolinium, 4,5-diethyl-1,3-dimethylimidazolinium, 3,4-diethyl-1,2-dimethylimidazolinium, 1,2,3-triethyl-4-methylimidazolinium, 1,2,4-triethyl-3-methylimidazolinium, 1,2,5-triethyl-3-methylimidazolinium, 1,3,4-triethyl-2-methylimidazolinium, 1,3,4-triethyl-5-methylimidazolinium, 1,4,5-triethyl-3-methylimidazolinium, 2,3,4-triethyl-1-methylimidazolinium, 4-cyano-1,2,3-trimethylimidazolinium, 3-cyanomethyl-1,2-dimethylimidazolinium, 2-cyanomethyl-1,3-dimethylimidazolinium, 4-acetyl-1,2,3-trimethylimidazolinium, 3-acetylmethyl-1,2-dimethylimidazolinium, 4-methylcarbo-oxymethyl-1,2,3-trimethylimidazolinium, 3-methylcarbo-oxymethyl-1,2-dimethylimidazolinium, 4-methoxy-1,2,3-trimethylimidazolinium, 3-methoxymethyl-1,2-dimethylimidazolinium, 4-formyl-1,2,3-trimethylimidazolinium, 3-formylmethyl-1,2-dimethylimidazolinium, 3-hydroxyethyl-1,2-dimethylimidazolinium, 4-hydroxymethyl-1,2,3-trimethylimidazolinium, 2-hydroxyethyl-1,3-dimethylimidazolinium and compounds in which a hydrogen atom at 2-position of the imidazolinium-based cations described above is replaced with a fluorine atom, and the like ;

### (Imidazolium cation)

1,3-dimethylimidazolium, 1-ethyl-3-methylimidazolium, 1-n-propyl-3-methylimidazolium, 1-n-butyl-3-methylimidazolium, 1,3-diethylimidazolium, 1,2,3-trimethylimidazolium, 1,2,3,4-tetramethylimidazolium, 1,3,4-trimethylimidazolium, 1,3,4-trimethyl-2-ethylimidazolium, 1,3-dimethyl-2,4-diethylimidazolium, 1,2-dimethyl-3,4-diethylimidazolium,1-methyl-2,3,4-triethyl imidazolium, 1,2,3,4-tetraethylimidazolium, 1,3-dimethyl-2-ethylimidazolium, 1-ethyl-2,3-dimethylimidazolium, 1-n-propyl-2,3-dimethylimidazolium, 1-n-butyl-2,3-dimethylimidazolium, 1,2,3-triethylimidazolium, 1,1-dimethyl-2-heptylimidazolium, 1,1-dimethyl-2-(2'-heptyl)imidazolium, 1,1-dimethyl-2-(3'-heptyl)imidazolium, 1,1-dimethyl-2-(4'-heptyl)imidazolium, 1,1-dimethyl-2-dodecylimidazolium, 1,1-dimethylimidazolium, 1,1,2-trimethylimidazolium, 1,1,2,4-tetramethylimidazolium, 1,1,2,5-tetramethylimidazolium, 1,1,2,4,5-pentamethylimidazolium, 1-ethyl-3-,4-dimethylimidazolium, 1-ethyl-3,5-dimethylimidazolium, 2-ethyl-1,3-dimethylimidazolium, 4-ethyl-1,3-dimethylimidazolium, 1,2-diethyl-3-methylimidazolium, 1,4-diethyl-3-methylimidazolium, 1,5-diethyl-3-methylimidazolium, 1,3-diethyl-2-methylimidazolium, 1,3-diethyl-4-methylimidazolium, 1,2,3-triethylimidazolium, 1,3,4-triethylimidazolium, 1-ethyl-2,3,4-trimethylimidazolium, 1-ethyl-2,3,5-trimethylimidazolium, 1-ethyl-3,4,5-trimethylimidazolium, 2-ethyl-1,3,4-trimethylimidazolium, 4-ethyl-1,2,3-trimethylimidazolium, 1,2-diethyl-3,4-dimethylimidazolium, 1,3-diethyl-2,4-dimethylimidazolium, 1,4-diethyl-2,3-dimethylimidazolium, 1,4-diethyl-2,5-dimethylimidazolium, 2,4-diethyl-1,3-dimethylimidazolium, 4,5-diethyl-1,3-dimethylimidazolium, 3,4-diethyl-1,2-dimethylimidazolium, 2,3,4-triethyl-1-methylimidazolium, 1,2,3-triethyl-4-methylimidazolium, 1,2,4-triethyl-3-methylimidazolium, 1,2,5-triethyl-3-methylimidazolium, 1,3,4-triethyl-2-methylimidazolium, 1,3,4-triethyl-5-methylimidazolium, 1,4,5-triethyl-3-methylimidazolium, 1,2,3,4-tetraethylimidazolium, 1,2,3,4,5-pentamethylimidazolium, 1-phenyl-3-methylimidazolium, 1-phenyl-3-ethylimidazolium, 1-benzyl-3-methylimidazolium, 1-benzyl-3-ethylimidazolium, 1-phenyl-2,3-dimethylimidazolium, 1-phenyl-2,3-diethylimidazolium, 1-phenyl-2-methyl-3-ethylimidazolium, 1-phenyl-2-ethyl-3-methylimidazolium, 1-benzyl-2,3-dimethylimidazolium, 1-benzyl-2,3-diethylimidazolium, 1-benzyl-2-methyl-3-ethylimidazolium, 1,3-dimethyl-2-phenylimidazolium, 1,3-diethyl-2-phenyl-imidazolium, 1-methyl-2-phenyl-3-ethylimidazolium, 1-methyl-2-phenyl-3-methylimidazolium, 1,3-dimethyl-2-benzyl-imidazolium, 1,3-diethyl-2-benzyl-imidazolium, 1,3-dimethyl-2-ethoxymethylimidazolium, 1,3-diethyl-2-ethoxymethylimidazolium, 1-methyl-2-ethoxymethyl-3-ethylimidazolium, 1-ethoxymethyl-2,3-dimethylimidazolium, 1-ethoxymethyl-2,3-diethylimidazolium, 1-ethoxymethyl-2-methyl-3-ethylimidazolium, 1,3-dimethyl-2-methoxymethylimidazolium, 1,3-diethyl-2-methoxymethylimidazolium, 1-methyl-2-methoxymethyl-3-ethylimidazolium, 1-methoxymethyl-2,3-dimethylimidazolium, 1-methoxymethyl-2,3-diethylimidazolium, 1-methoxymethyl-2-methyl-3-ethylimidazolium, 1,3-dimethyl-2-methoxyethylimidazolium, 1,3-diethyl-2-methoxyethylimidazolium, 1-methyl-2-methoxyethyl-3-ethylimidazolium, 1-methoxyethyl-2,3-dimethylimidazolium, 1-methoxymethyl-2,3-diethylimidazolium, 1-methoxyethyl-2-methyl-3-ethylimidazolium, 1,3-dimethylbenzoimidazolium, 1,3-diethylbenzoimidazolium, 1-methyl-3-ethylbenzoimidazolium, 1,2,3-trimethylbenzoimidazolium, 1,2-dimethyl-3-ethylbenzoimidazolium, 2-cyanomethyl-1,3-dimethylimidazolium, 4-acetyl-1,2,3-trimethylimidazolium, 3-acetylmethyl-1,2-dimethylimidazolium, 4-methylcarboxymethyl-1,2,3-trimethylimidazolium, 3-methylcarboxymethyl-1,2-dimethylimidazolium, 4-methoxy-1,2,3-trimethylimidazolium, 3-methoxymethyl-1,2-dimethylimidazolium, 4-formyl-1,2,3-trimethylimidazolium, 3-formylmethyl-1,2-dimethylimidazolium, 3-hydroxyethyl-1,2-dimethylimidazolium, 4-hydroxymethyl-1,2,3-trimethylimidazolium, 2-hydroxyethyl-1,3-dimethylimidazolium, and cations obtained by substituting a hydrogen atom at 2-position of the above-described imidazolinium cations by a fluorine atom, and the like.

### (Quinolinium cation)

N-methylquinolinium, N-ethylquinoliniumand and the like;

### (Bipyridinium cation)

It represents an ion such as N-methyl-2,2'-bipyridinium, N-ethyl-2,2'-bipyridinium and the like;

### (Other ammonium cations)

N-methylthiazolium, N-ethylthiazolium, N-methyloxazolium, N-ethyloxazolium, N-methyl-4-methylthiazolium, N-ethyl-4-methylthiazolium, N-ethylisothiazolium, 1,4-dimethyl-1,2,4-triazolium, 1,4-diethyl-1,2,4-triazolium, 1-methyl-4-ethyl-1,2,4-triazolium, 1-ethyl-4-methyl-1,2,4-triazolium, 1,2-dimethylpyrazolium, 1,2-diethylpyrazolium, 1-methyl-2-ethylpyrazolium, N-methylpyrazinium, N-ethylpyrazinium, N-methylpyridazinium, N-ethylpyridazinium and the like are listed;

### (Tetraalkylphosphonium cations)

tetramethylphosphonium, ethyltrimethylphosphonium, triethylmethylphosphonium, tetraethylphosphonium, diethyldimethylphosphonium, trimethyl-n-propylphosphonium, trimethylisopropylphosphonium, ethyldimethyl-n-propylphosphonium, ethyldimethylisopropylphosphonium, diethylmethyl-n-propylphosphonium, diethylmethylisopropylphosphonium, dimethyldi-n-propylphosphonium, dimethyl-n-propylisopropylphosphonium, dimethyldiisopropylphosphonium, triethyl-n-propylphosphonium, n-butyltrimethylphosphonium, isobutyltrimethylphosphonium, t-butyltrimethylphosphonium, triethylisopropylphosphonium, ethylmethyldi-n-propylphosphonium, ethylmethyl-n-propylisopropylphosphonium, ethylmethyldiisopropylphosphonium, n-butylethyldimethylphosphonium, isobutylethyldimethylphosphonium, t-butylethyldimethylphosphonium, diethyldi-n-propylphosphonium, diethyl-n-propylisopropylphosphonium, diethyldiisopropylisopropylphosphonium, methyltri-n-propylphosphonium, methyldi-n-propylisopropylphosphonium, methyl-n-propyldiisopropylphosphonium, n-butyltriethylphosphonium, isobutyltriethylphosphonium, t-butyltriethylphosphonium, di-n-butyldimethylphosphonium, diisobutyldimethylphosphonium, di-t-butyldimethylphosphonium, n-butylisobutyldimethylphosphonium, n-butyl-t-butyldimethylphosphonium, isobutyl-t-butyldimethylphosphonium, tri-n-octylmethylphosphonium, ethyltri-n-octylphosphonium and the like.

As the organic cation, organic quaternary ammonium cations are preferable among them, and imidazolium cations are preferable among them, and 1-ethyl-3-methylimidazolium (EMI⁺) represented by the formula (4): 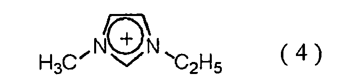 is especially preferable, because of a tendency of increase in electrostatic capacity per unit volume.

The organic anion is an anion containing a hydrocarbon group optionally having substituent, andexamples thereof include an anion selected from the group consisting of N(SO₂R_{f})²⁻, C(SO₂R_{f})³⁻, R_{f}COO⁻ and R_{f}SO³⁻ (wherein R_{f} represents perfluoroalkyl group having 1 to 12 carbon atoms) and anions formed by eliminating an active hydrogen from the following organic acids (carboxylic acids, organic sulfonic acids and organic phosphoric acids) or phenols.

### (Carboxylic acids)

· two to four valent polycarboxylic acids having 2 to 15 carbon atoms: aliphatic polycarboxylic acids [saturated polycarboxylic acids (oxalic acid, malonic acid, succinic acid, glutaric acid, adipic acid, pimelic acid, suberic acid, azelaic acid, sebacic acid, undecanedioic acid, dodecanedioic acid, tridecanedioic acid, tetradecanedioic acid, pentadecanedioic acid, hexadecanedioic acid, methylmalonic acid, ethylmalonic acid, propylmalonic acid, butylmalonic acid, pentylmalonic acid, hexylmalonic acid, dimethylmalonic acid, diethylmalonic acid, methylpropylmalonic acid, methylbutylmalonic acid, ethylpropylmalonic acid, dipropylmalonic acid, methylsuccinic acid, ethylsuccinic acid, 2,2-dimethylsuccinic acid, 2,3-dimethylsuccinic acid, 2-methylglutaric acid, 3-methylglutaric acid, 3-methyl-3-ethylglutaric acid, 3,3-diethylglutaric acid, methylsuccinic acid, 2-methylglutaric acid, 3-methylglutaric acid, 3, 3-dimethylglutaric acid, 3-methyladipic acid and the like), and unsaturated polycarboxylic acids (cyclobutene-1,2-dicarboxylic acid, 4-methylcyclobutene-1,2-dicarboxylic acid, cyclopentene-1,2-dicarboxylic acid, 5-methyl-cyclopentene-1,2-dicarboxylic acid, bicyclo[2,2,1]hepta-2-en-2,3-dicarboxylic acid, 1-methyl-bicyclo[2,2,1]hepta-2-en-2,3-dicarboxylic acid, 6-methyl-bicyclo[2,2,1]hepta-2-en-2,3-dicarboxylic acid, bicyclo[2,2,1]hepta-2,5-diene-2,3-dicarboxylic acid, 1-methyl-bicyclo[2,2,1]hepta-2,5-diene-2,3-dicarboxylic acid, 6-methyl-bicyclo[2,2,1]hepta-2,5-diene-2,3-dicarboxylic acid, furan-2,3-dicarboxylic acid, 5-methyl-furan-2,3-dicarboxylic acid, 4-methyl-furan-2,3-dicarboxylic acid, 4,5-dihydroxy-furan-2,3-dicarboxylic acid, 4,5-dihydroxy-4-methyl-furan-2,3-dicarboxylic acid, 4,5-dihydroxy-5-methyl-furan-2,3-dicarboxylic acid, 2,5-dihydroxy-furan-3,4-dicarboxylic acid, 2,5-dihydroxy-2-methyl-furan-3,4-dicarboxylic acid and the like. Among them, preferable ones are cyclobutene-1,2-dicarboxylic acid, 4-methyl-cyclobutene-1,2-dicarboxylic acid, cyclopentene-1,2-dicarboxylic acid, 5-methyl-cyclopentene-1,2-dicarboxylic acid, bicyclo[2,2,1]hepta-2-en-2,3-dicarboxylic acid, bicyclo[2,2,1]hepta-2,5-diene-2,3-dicarboxylic acid, furan-2,3-dicarboxylic acid, 5-methyl-furan-2,3-dicarboxylic acid, 4-methyl-furan-2,3-dicarboxylic acid, 5-methyl-2,3-furandicarboxylic acid, 4,5-dihydroxy-furan-2,3-dicarboxylic acid, 2,5-dihydroxy-furan-3,4-dicarboxylic acid, maleic acid, fumaric acid, itaconic acid, citraconic acid, 1,2-cyclobutadiene-1,2-dicarboxylic acid, 4-methyl-1,2-cyclobutadiene-1,2-dicarboxylic acid, 1,2-cyclopentadiene-1,2-dicarboxylic acid, 5-methyl-1,2-cyclopentadiene-1,2-dicarboxylic acid, 1,2-cyclohexadiene-1,2-dicarboxylic acid, 6-methyl-1,2-cyclohexadiene-1,2-dicarboxylic acid, 5-methyl-1,2-cyclohexadiene-1,2-dicarboxylic acid, furan-3,4-dicarboxylic acid and 2-methyl-furan-3,4-dicarboxylic acid. Among them, preferable ones are 1,2-cyclobutadiene-1,2-dicarboxylic acid, 4-methyl-1,2-cyclobutadiene-1,2-dicarboxylic acid, 1,2-cyclopentadiene-1,2-dicarboxylic acid, 5-methyl-1,2-cyclopentadiene-1,2-dicarboxylic acid, furan-3,4-dicarboxylic acid and 2-methyl-3,4-furandicarboxylic acid)], aromatic polycarboxylic acids [phthalic acid, isophthalic acid, terephthalic acid, trimellitic acid, pyromellitic acid and the like] and polycarboxylic acids containing sulfur atom [thiodipropionic acid and the like];

· oxycarboxylic acids having 2 to 20 carbon atoms: aliphatic oxycarboxylic acids [glycolic acid, lactic acid, tartaric acid, castor oil fatty acid and the like] ; and aromatic oxycarboxylic acids [salicylic acid, mandelic acid, 4-hydroxybenzoic acid, 1-hydroxy-2-naphthoic acid, 3-hydroxy-2-naphthoic acid, 6-hydroxy-2-naphthoic acid and the like]; · monocarboxylic acids having 1 to 30 carbon atoms: aliphatic monocarboxylic acids [saturated monocarboxylic acids (formic acid, acetic acid, propionic acid, butyric acid, isobutyric acid, valeric acid, capric acid, enanthic acid, capric acid, pelargonic acid, lauric acid, myristic acid, stearic acid, behenic acid, undecanoic acid and the like), unsaturated monocarboxylic acids (acrylic acid, methacrylic acid, crotonic acid, oleic acid, squaric acid, 4,5-dihydroxy-4-cyclopentene-1,3-dione, 2,3-dihydroxy-2-cyclohexene-1,4-dione and the like)]; aromatic monocarboxylic acids [benzoic acid, cinnamic acid, naphthoic acid, toluic acid, ethylbenzoic acid, propylbenzoic acid, isopropylbenzoic acid, butylbenzoic acid, isobutylbenzoic acid, secondary-butylbenzoic acid, tertiary-butylbenzoic acid, hydroxybenzoic acid, ethoxybenzoic acid, propoxybenzoic acid, isopropoxybenzoic acid, butoxybenzoic acid, isobutoxybenzoic acid, secondary-butoxybenzoic acid, tertiary-butoxybenzoic acid, aminobenzoic acid, N-methylaminobenzoic acid, N-ethylaminobenzoic acid, N-propylaminobenzoic acid, N-isopropylaminobenzoic acid, N-butylaminobenzoic acid, N-isobutylaminobenzoic acid, N-secondary-butylaminobenzoic acid, N-tertiary-butylaminobenzoic acid, N,N-dimethylaminobenzoic acid, N,N-diethylaminobenzoic acid, nitrobenzoic acid, florobenzoic acid and the like]

### (Phenols)

· monophenols (including phenols and naphthols): phenol, alkyl(having 1 to 15 carbon atoms) phenols (cresol, xylenol, ethylphenol, n- or iso-propylphenol, isododecylphenol and the like) , methoxyphenols (eugenol, guaiacol and the like), α-naphthol, β-naphthol, cyclohexylphenol and the like; · polyphenols: catechol, resorcinol, pyrogallol, phloroglucine, bisphenol A, bisphenol F, bisphenol S and the like

### (Phosphates having one or two alkyl groups having 1 to 15 carbon atoms in its molecule)

mono- and di-methylphosphate, mono- and di-isopropylphosphate, mono- and di-butylphosphate, mono- and di-(2-ethylhexyl)phosphate, mono- and di-isodecylphosphate and the like

### (Organic sulfonic acids)

alkyl(having 1 to 15 carbon atoms)benzenesulfonic acids (p-toluenesulfonic acid, nonylbenzenesulfonic acid, dodecylbenzenesulfonic acid and the like), sulfosalicylic acid, methanesulfonic acid, trifloromethanesulfonic acid and the like

### (Organic acids having a triazole or tetrazole structure)

1-H-1,2,4-triazole, 1,2,3-triazole, 1,2,3-benzotriazole, carboxybenzotriazole, 3-mercapto-1,2,4-triazole, 1,2,3-triazole-4,5-dicarboxylic acid, 3-mercapto-5-methyl-1,2,4-triazole, 1,2,3,4-tetrazole and the like

### (Boron-containing organic acids)

borodioxalate, borodiglycolate, borodi(2-hydroxyisobutyrate), alkane borates, aryl borates, methane borate, ethane borate, phenyl borates and the like

Anions represented by the following formula:

[(R_{f})ₖBF₄₋ₖ]⁻

wherein k represents an integer of 1 to 4 and R_{f} represents the same meaning mentioned above.
trifluoromethyltrifluoro borate, bis(trifluoromethyl)difluoro borate, tris(trifluoromethyl)fluoro borate, tetrakis(trifluoromethyl) borate, pentafluoroethyltrifluoro borate, bis(pentafluoroethyl)difluoro borate, tris(pentafluoroethyl)fluoro borate, tetrakis(pentafluoroethyl) borate and the like

Anions represented by the following formula: 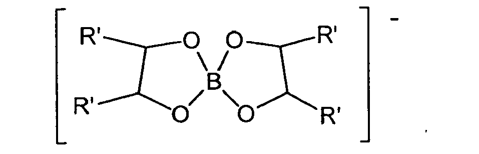 wherein R' represents a hydrocarbon group having 1 to 10 carbon atoms which may have a hydroxyl group, an amino group, a nitro group, a cyano group, a chloro group, a fluoro group, a formyl group or a group having an ether bonding, or hydrogen atom or fluorine atom, and R's may be same or different from each other, and R's may be bonded with each other together with an alkylene group to form a ring.

Anions represented by the following formula: 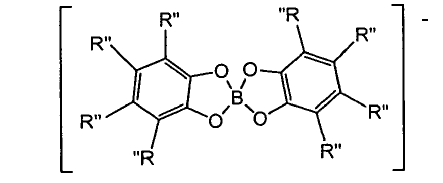 wherein R'' represents the same meaning as R', and R''s may be same or different from each other, and R''s may be bonded with each other together with a hydrocarbon group to form a ring.

Anions represented by the following formulae: 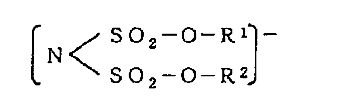 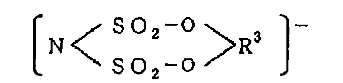 wherein R¹ and R² are monovalent organic groups having 1 to 4 carbon atoms and a fluorine atom, and R¹ and R² may be same or different from each other, and R³ is a divalent organic group having 2 to 8 carbon atoms and a fluorine atom.

As the anion, an inorganic anion is preferable, and especially, BF₄⁻, AsF₆⁻ and SbF₆⁻ are more preferable, and among them, BF₄⁻ is especially preferable in viewpoint of enhancing electrostatic capacity.

Examples of the solvent used for the electrolytic solution containing an electrolyte include water and/or organic polar solvents. As the electrolytic solution containing an inorganic electrolyte, solvents mainly composed of water are usually used, and the hydrophilic organic solvents listed above may also be used together with water. As the electrolytic solution containing an organic electrolyte, solvents mainly composed of an organic polar solvent are used, and the content of water contained in the electrolytic solution containing an organic polar solvent is usually 200 ppm or less, preferably 50 ppm or less, and more preferably 20 ppm or less. By suppressing the content of water in the electrolytic solution containing an organic polar solvent, influences on an electrode by electrolysis of water, particularly, lowering of withstand voltage, can be suppressed.

Herein, specific examples of the organic polar solvents include the followings:

### (Ethers)

monoethers (ethyleneglycol monomethylether, ethyleneglycol monoethylether, diethyleneglycol monomethylether, diethyleneglycol monoethylether, ethyleneglycol monophenylether, tetrahydrofuran
3-methyltetrahydrofuran, and the like), diethers (ethyleneglycol dimethylether, ethyleneglycol diethylether, diethyleneglycol dimethylether, diethyleneglycol diethylether, diethylether, methylisopropylether, and the like), triethyleneglycol dimethylether, ethyleneglycol monomethylether acetate, cyclic ethers [having 2 to 4 carbon atoms (tetrahydrofuran, 2-methyltetrahydrofuran, 1,3-dioxolane, 1,4-dioxane, 2-methyl-1,3-dioxolane, and the like); 4-butyldioxolane and crown ether having 5 to 18 carbon atoms] an the like

### (Fluorinated dioxolanes)

2,2-di(trifluoromethyl)-1,3-dioxolane, 2,2-di(trifluoromethyl)-4,5-difluoro-1,3-dioxolane, 2,2-di(trifluoromethyl)-4,4,5,5-tetrafluoro-1,3-dioxolane, 2,2-dimethyl-4,4,5,5-tetrafluoro-1,3-dioxolane or 2,2-dimethyl-4,5-difluoro-1,3-dioxolane

### (Amides)

formamides (N-methylformamide, N,N-dimethylformamide, N-ethylformamide, N,N-diethylformamide, and the like), acetamides (N-methylacetamide, N,N-dimethylacetamide, N-ethylacetamide, N,N-diethylacetamide, and the like), propionamides (N,N-dimethylpropionamide and the like), hexamethylphosphorylamide and the like, oxazolidinones ; N-methyl-2-oxazolidinone, 3,5-dimethyl-2-oxazolidinone and the like, 1,3-dimethyl-2-imidazolidinone, N-methylpyrrolidone and the like

### (Nitriles)

acetonitrile, glutaronitrile, adiponitrile, methoxyacetonitrile, 3-methoxypropionitrile, acrylonitrile and fluorine-containing propionitriles in which one or more hydrogen atoms of propionitrile are substituted with fluorine atom(s) and the like

### (Carboxylates)

methyl formate, ethyl formate, methyl acetate, ethyl acetate, propyl acetate, methyl propionate, methyl butyrate, methyl valerate, ethyl propionate, dimethyl malonate, diethyl malonate and the like, maleic anhydride and derivatives thereof,

### (Lactones)

γ-butyrolactone, 3-methyl-γ-butyrolactone, 2-methyl-γ-butyrolactone, α-acetyl-γ-butyrolactone, β-butyrolactone, γ-valerolactone, 3-methyl-γ-valerolactone, δ-valerolactone and the like

### (Carbonates)

ethylene carbonate, propylene carbonate, butylene carbonate, vinylene carbonate, dimethylcarbonate, methylethylcarbonate, methylpropylcarbonate, methylisopropylcarbonate, diethylcarbonate, 4-allyloxymethyl-1,3-dioxolane-2-one, 4-(1'-propenyloxymethyl)-1,3-dioxolane-2-one, 4-allyloxymethyl-5-vinyl-1,3-dioxolane-2-one, 4-(1'-propenyloxymethyl)-5-vinyl-1,3-dioxolane-2-one, 4-acryloyloxymethyl-1,3-dioxolane-2-one, 4-methacryloyloxymethyl-1,3-dioxolane-2-one, 4-methacryloyloxymethyl-5-vinyl-1,3-dioxolane-2-one, 4-methoxycarbonyloxymethyl-1,3-dioxolane-2-one, 4-allyloxycarbonyloxymethyl-1,3-dioxolane-2-one, 4-(1'-propenyloxycarbonyloxymethyl)-1,3-dioxolane-2-one, 4-vinylethylene carbonate, 4,5-divinylethylene carbonate, 4,4,5,5-tetramethyl-1,3-dioxolane-2-one, 4,4,5,5-tetraethyl-1,3-dioxolane-2-one, vinylene carbonate, 4-methylvinylene carbonate, 4,5-dimethylvinylene carbonate, 5,5-dimethyl-1,3-dioxane-2-one and 5,5-diethyl-1,3-dioxane-2-one, dipropylcarbonate, methylbutylcarbonate, ethylbutylcarbonate, ethylpropylcarbonate, butylpropylcarbonate, and compounds of which one or more hydrogen atoms are substituted with fluorine atom(s) and the like

### (Sulfoxides)

dimethylsulfoxide, sulfolane, 3-methylsulfolane, 2,4-dimethylsulfolane and fluorine-containing sulfolanes in which one or more hydrogen atoms of sulfolane are substituted with fluorine atom(s), and the like 1,3-propanesultone, 1,4-butanesultone, and compounds of which one or more hydrogen atoms are substituted with fluorine atom(s), and the like

### (Sulfone)

dimethylsulfone, diethylsulfone, di-n-propylsulfone, di-isopropylsulfone, di-n-butylsulfone, di-sec-butylsulfone, di-tert-butylsulfone and the like

### (Nitro compounds)

nitromethane, nitroethane and the like

### (Other heterocyclic compounds)

N-methyl-2-oxazolidinone, 3,5-dimethyl-2-oxazolidinone, 1,3-dimethyl-2-imidazolidinone, N-methylpyrrolizinone and the like

### (Monohydric alcohols)

monohydric alcohols having 1 to 6 carbon atoms (methyl alcohol, ethyl alcohol, propyl alcohol, butyl alcohol, diacetone alcohol, furfuryl alcohol and the like), and monohydric alcohols having 7 carbon atoms or more (benzyl alcohol, octyl alcohol and the like)

### (Polyhydric alcohols)

dihydric alcohols having 1 to 6 carbon atoms (ethyleneglycol, propyleneglycol, diethyleneglycol, hexyleneglycol and the like), dihydric alcohols having 7 carbon atoms or more (octyleneglycol and the like), trihydric alcohols (glycerin and the like), hexahydric alcohols (hexytol and the like) and the like

### (Hydrocabons)

aromatic solvents (toluene, xylene, ethylfluorobenzene, fluorobenzenes in which 1 to 6 hydrogen atoms of benzene are substituted with fluorine atom(s) and the like), paraffinic solvents (normal paraffin, isoparaffin and the like) and the like

### (Silicon compounds)

oxazolidinone compounds such as 3-trimethylsilyl-2-oxazolidinone, 3-trimethylsilyl-4-trifluoromethyl-2-oxazolidinone and 3-triethylsilyl-2-oxazolidinone, imidazole compounds such as N-trimethylsilylimidazole, N-trimethylsilyl-4-methylimidazole and N-triethylsilylimidazole, phosphate compounds such as tris(trimethylsilyl)phosphate, tris(triethylsilyl)phosphate, trimethylsilyl dimethylphosphate and trimethylsilyl diallylphosphate, cyclic carbonates such as 4-trimethylsilyl-1,3-dioxolane-2-one, 4-trimethylsilyl-5-vinyl-1,3-dioxolane-2-one and 4-trimethylsilylmethyl-1,3-dioxolane-2-one, phenyl compounds such as phenyltrimethylsilane, phenyltriethylsilane, phenyltrimethoxysilane, phenylthiotrimethylsilane and phenylthiotriethylsilane, carbamate compounds such as methyl-N-trimethylsilylcarbamate, methyl-N,N-bistrimethylsilyl carbamate, ethyl-N-trimethylsilyl carbamate, methyl-N-triethylsilyl carbamate and vinyl-N-trimethylsilyl carbamate, carbonate compounds such as methyltrimethylsilyl carbonate, allyltrimethylsilyl carbonate and ethyltrimethylsilyl carbonate, methoxytrimethylsilane, hexamethyldisiloxane, pentamethyldisiloxane, methoxymethyltrimethylsilane, trimethylchlorosilane, butyldiphenylchlorosilane, trifluoromethyltrimethylsilane, acetyltrimethylsilane, 3-trimethylsilylcyclopentene, allyltrimethylsilane, vinyltrimethylsilane, hexamethyldisilazane and the like, having silicon atom(s) in its molecule

The organic polar solvents dissolving the electrolytes may be a mixture of two or more different kinds thereof.

The organic polar solvent contained in the electrolytic solution is preferably a solvent mainly containing at least one selected from the group consisting of carbonates, lactones, and sulfoxides, more preferably a solvent mainly containing at least one selected from the group consisting of propylene carbonate, ethylene carbonate, butylene carbonate, sulfolane, 3-methylsulfolane, acetonitrile, dimethyl carbonate, ethyl methyl carbonate, γ-butyrolactone, ethylene glycol, and diethyl carbonate, and further more preferably a solvent mainly containing at least one selected from the group consisting of ethylene carbonate, propylene carbonate, γ-butyrolactone, and sulfolane. Herein, the term "mainly containing" defines that the compound(s) occupies 50% by weight or more and preferably 70% by weight or more of the weight of the solvent. The higher the content of the organic polar solvent is, the more long-term durability and operating voltage of the capacitor can be enhanced.

To the electrolytic solution, various additives can be added, if necessary. Specifc examples thereof include phosphates (trimethyl phosphate, triethyl phosphate, triallyl phosphate and the like), phosphonic acid and the like] for suppressing gas generation and enhancing voltage endurance, and fluorine-containing organic silicon compounds represented by the following fomula:

CF₃CH₂CH₂Si(CH₃)₃,

(CH₃)₃Si-O-Si(CH₃)(CF₃CH₂CH₂)-Si(CH₃)

for achieving high capacity and output.

From the viewpoint of electric conductivity of the electrolyte and solubility to the solvent of the electrolytic solution, the additive amount of the phosphate is usually about 10% by weight or less of the electrolyte, and the additive amount of the fluorine-containing organic silicon compound is about 0.1 to 5% by weight in the electrolytic solution.

Benzoic acids [for example, alkyl benzoates such as methyl benzoate, ethyl benzoate and propyl benzoate, benzoic acid and the like], being one kind of organic polar solvents, may be used as an additive for preventing metal elution from current collector. When the benzoic acid is used as an additive, it is used usually about 0.001 to 10.0% by weight of the electrolyte, preferably 0.005 to 5% by weight, and more preferably 0.1 to 1% by weight.

Concentration of the organic electrolyte in the electrolytic solution containing the organic electrollte is usually about 0.5 to 5.0 moles (electrolyte)/L (electrolytic solution) and preferably about 0.7 to 3.0 moles (electrolyte) /L (electrolytic solution). Dissolving the electrolyte in 0.5 mol/L or more is preferable since electrostatic capacity tends to increase, and dissolving the electrolyte in 5. 0 mol/L or less is prefersble, since viscosity tends to decrease.

The electric double-layer capacitor is usually charged by applying current of about 5 mA/g to 10 A/g and preferably 10 mA/g to 5 A/g. Applying the current of 5 mA/g or more is preferable since charging rate tends to be enhanced, and applying the current of 10 A/g or less is preferable since reduction of electrostatic capacity tends to be suppressed.

Alternatively, the electric double-layer capacitor containing the activated carbon of the present invention causes little reduction of electrostatic capacity even if being repeatedly subjected to quick charging/discharging at a current of 1 A/g or more.

The electric double-layer capacitor containing the activated carbon of the present invention is an electric double-layer capacitor excellent in electrostatic capacity, having an electrostatic capacity per unit volume of the activated carbon of usually 25 F/ml or more and preferably 30 F/ml or more. Alternatively, it also has an excellent electric property wherein the electrostatic capacity per unit weight of the activated carbon is usually 25 F/g or more and preferably 30 F/g or more.

Meanwhile, the electrostatic capacity of the activated carbon can be obtained by followings: A mixture of 80 parts by weight of an activated carbon and 10 parts by weight (solid component) of polytetrafluoroethylene is kneaded, then, put into a vessel having diameter of 13 mm, molded by pressing under 162 kgf/cm², and the density (g/cc) of the molded article is measured, and the electrostatic capacity per unit weight of the molded article is obtained from a discharge curve in discharging after charging up to 2.8 V with a constant current of 300 mA/g with a charge and discharge evaluation apparatus TOSCAT-3100 manufactured by TOYO System K.K., and the electrostatic capacity per unit volume is calculated by multiplying the resultant electrostatic capacity per unit weight by the density obtained above.

Further, the electric double-layer capacitor containing the activated carbon of the present invention has an excellent electric property that if the electrostatic capacity in charging and discharging with constant current (300 mA, 0 to 2.3 V) is defined to 100, lowering of electrostatic capacity is small even if quick charging and discharging is carried out, and if the electrolytic solution is a 3 mol/L solution of 1-ethyl-3-methylimidazolium BF₄ salt in propylene carbonate, lowering of electrostatic capacity is usually less than 5% and preferably 3% or less even if quick charging and discharging with 4000 mA/g is carried out.

When the activated carbon of the present invention is used as an electric double-layer capacitor, the electrostatic capacity per unit volume or electrostatic capacity per unit weight is improved remarkably as compared with the case of use of any of an activated carbon obtained by carbonizing and activating an organic aerogel and a cyclic tetramer carbide of resorcinol and an aldehyde compound. Lternatively, even if quick charging and discharging is repeated, preserved electric quantity scarcely decrease.

### Examples

The present invention will be illustrated in more detail based on Examples, but it is needless to say that the present invention is not limited to these Examples.

### (Synthesis Example 1 of compound (1))

### Production of tetramethylcalix[4]resocrinalene (compound (1): MCRA)

Into a four-necked flask, 30.0 g of resorcinol, 120 ml of ethanol and 12.1 g of acetaldehyde were added under nitrogen flow to cool with ice, and 53.7 g of 36% hydrochloric acid was dropped thereto with stirring. After completion of dropping, the temperature was raised to 65°C, and then, the mixture was kept at the same temperature for 5 hours. To the obtained reaction mixture, 320 g of water was added, and the produced precipitate was taken out by filtration, and washed with water until the filtrate became neutral, and dried, then, recrystallized from a mixed solvent of water-ethanol to obtain 13.1 g of tetramethylcalix[4]resocrinalene (MCRA).
Mass Analysis Value of MCRA (FD-MS) m/z 544
¹H NMR of MCRA (DMSO-d⁶) : δ1.29 (s, 12H), 4.45 (q, 4H), 6.14 (s, 4H), 6.77 (s, 4H), 8.53 (s, 8H)

### (Example 1)

### (Preparation of activated carbon)

An aqueous 10 wt% potassium hydroxide solution and MCRA were mixed so that the amount of potassium hydroxide (solid component) would be 1.5 parts by weight per 1 part by weight of MCRA, and moisture was removed by a vacuum drying machine, and subsequently, an activation treatment was carried out at 800°C for 4 hours under an argon atmosphere, allowed to cool to ambient temperature and then, added 0.1 mol/L dilute hydrochloric acid thereto to wash. An activated carbon was separated by filtration and ion exchanged water was added thereto to wash. Filtration and drying were sequentially conducted to obtain an activated carbon. The activated carbon after drying was, then, ground with a ball mill (ball made of agate, 28 rpm, 5 minutes).

It was caluculated that the resultant activated carbon had a total pore volume of 0.89 ml/g and a micro pore volume of 0.84 ml/g, and the meso pore volume of 0.05 ml/g.

Here, the total pore volume is calculated from nitrogen adsorption amount around a relative pressure of 0.95 in a nitrogen adsorption isothermal curve at liquid nitrogen temperature using AUTOSORB manufactured by YUASA IONICS, and the micro pore volume is calculated from around a relative pressure of 0.30.

### (Production Example of electrode and electric double-layer capacitor)

A mixture of 80 parts by weight of the above-described activated carbon, 10 parts by weight of acetylene black (manufactured by DENKA, Denka Black 50%, pressed product) and 10 parts by weight (solid component) of polytetrafluoroethylene (approximately 60 wt%-containing aqueous dispersion) was kneaded, then, molded into sheets of 0.28 mm, and dried to obtain electrodes. Between two resultant electrodes, cellulose for condenser (thickness 50 µm) was inserted as a separator, then, a bipolar electric double-layer capacitor (Fig. 5) filled with a 1 mol/L solution of tetraethylammonium BF₄ salt (organic electrolyte) in propylene carbonate was produced.

Accroding to the measurement of charge and discharge with constant current (300 mAg, 0 to 2.8 V) using the capacitor, an electrostatic capacity per unit volume of activated carbon was 28.1 F/cc and an electrostatic capacity per unit weight of activated carbon was 43.0 F/g.

Meanwhile, the electrostatic capacity of activated carbon was obtained as follows: A mixture of 80 parts by weight of the activated carbon used and 10 parts by weight (solid component) of polytetrafluoroethylene (approximately 60 wt%-containing aqueous dispersion) was kneaded, then, put into a vessel having diameter of 13 mm, molded by pressing under 162 kgf/cm², and the density (g/cc) of the molded article was measured, subsequently, the electrostatic capacity per unit weight of the molded article was obtained from a discharge curve in discharging after charging up to 2.8 V with a constant current of 300 mA/g in a charge and discharge evaluation apparatus TOSCAT-3100 manufactured by TOYO System K.K., and the electrostatic capacity per unit volume was calculated by multiplying the electrostatic capacity per unit weight by the density obtained above. The same procedure applies to the followings.

### (Examples 2 to 8)

These examples were carried out according to Example 1 excepting that the kind of the compound (1) was changed as described in Table 1. The results are shown in Table 1 together with Example 1.

**Table 1**

| Example | Compound (1) | | Total pore volume (ml/g) | Micro pore volume (ml/g) | Electrostatic capacity | | Filling density (g/ml) |
|---|---|---|---|---|---|---|---|
| | n | R | | | (F/ml) | (F/g) | |
| 1 | 4 | -CH₃ | 0.89 | 0.84 | 28.1 | 43.0 | 0.65 |
| 2 | 4 | 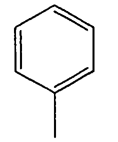 | 0.89 | 0.85 | 28.4 | 42.5 | 0.67 |
| 3 | 4 | 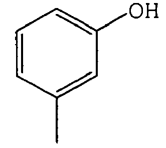 | 1.08 | 0.85 | 29.0 | 40.3 | 0.66 |
| 4 | 4 | 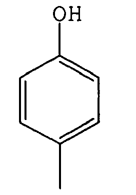 | 0.91 | 0.87 | 25.9 | 41.1 | 0.63 |
| 5 | 4 | 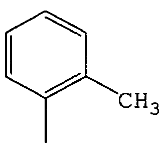 | 0.90 | 0.86 | 27.8 | 43.1 | 0.65 |
| 6 | 4 | 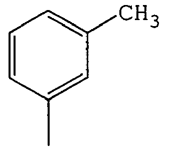 | 0.81 | 0.75 | 27.3 | 41.4 | 0.66 |
| 7 | 4 | 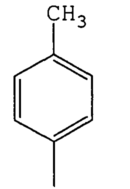 | 0.94 | 0.88 | 29.9 | 46.3 | 0.65 |
| 8 | 4 | 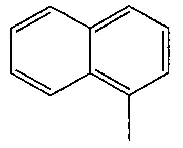 | 0.84 | 0.73 | 30.0 | 41.7 | 0.72 |

### (Example 9: Production Example using sodium hydroxide as activating agent)

This example was carried out according to Example 1 excepting that the kind of the compound (1) was changed to a compound described in Example 2 and an alkali metal hydroxide was changed to sodium hydroxide.

Accroding to the measurement of charge and discharge with constant current (300 mAg, 0 to 2.8 V) using the capacitor, a bulk density of activated carbon was 0. 70 g/ml, an electrostatic capacity per unit volume of activated carbon was 26.2 F/cc and an electrostatic capacity per unit weight of activated carbon was 37.2 F/g.

### (Example 10: Production Example using potassium hydroxide as activating agent for carbide of compound (1))

This example was carried out according to Example 1 excepting that a 30 wt% potassium hydroxide aqueous solution and MCRA were mixed wherein a carbide obtained by heating the compound described in Example 2 up to 700°C under a nitrogen atmosphere so that the weight ratio of potassium hydroxide (solid component)/carbide would be 4.0.

Accroding to the measurement of charge and discharge with constant current (300 mAg, 0 to 2.8 V) using the capacitor, a bulk density of activated carbon was 0. 65 g/ml, an electrostatic capacity per unit volume of activated carbon was 27.1 F/cc and an electrostatic capacity per unit weight of activated carbon was 41.4 F/g.

### (Comparative Examples 1 and 2)

Comparative Example 1 was carried out according to Example 1 excepting that the kind of the compound (1) was changed to that which is described in Example 2 and potassium hydroxide was not mixed. The results are shown in Table 2.

Comparative Example 2 was carried out according to Example 1 excepting that zinc chloride was used instead of potassium hydroxide and a 15 wt% zinc chloride aqueous solution was mixed so that the weight ratio of zinc chloride/carbide would be 2. The results are shown in Table 2.

### (Comparative Example 3)

In Comparative Example 3, those calculated according to the following calculation from Example 3 described in the patent document 1, was used as a comparison. Since the electrode in the form of disk according to the patent document 1 has a diameter of 10 mm and a thickness of 0.5 mm, the volume of the electrode is π × 5 mm × 5 mm × 0.5 mm = 39.25 mm³. According to Example 3 of the patent document 1, the weight of the electrode is 39.25 mm³ × 0.46 g/ml = 18.06 mg, and the capacity at 1 mA is 0.43 F, thus, the electrostatic capacity per unit weight is 0.43 F/18.06 × 10³ = 23.8 F/g, and the electrostatic capacity per unit volume is 23.8 F/g × 0.46 g/ml = 10.9 F/ml.

**Table 2**

| Comparative Example | Total pore volume | Micro pore volume | Current value | Electrostatic capacity | | Filling density |
|---|---|---|---|---|---|---|
| | (ml/g) | (ml/g) | (mA/g) | (F/ml) | (F/g) | (g/ml) |
| 1 | 0.35 | 0.2 | 300 | 1.1 | 0.9 | 1.2 |
| 2 | 0.65 | 0.59 | 300 | 19.6 | 25.6 | 0.77 |
| 3 | No description | No description | 1 | 10.9 | 23.8 | 0.46 |

### Industrial Applicability

The activated carbon of the present invention can be used for, for example, electrodes for dry batteries, sensors for piezoelectric devices, carries for supporting a catalyst, chromatograph materials, adsorbing agents, electrodes of electric double-layer capacitors, and the like, and, because of excellent electrostatic capacity per unit volume, is used suitably for electrodes of electric double-layer capacitors.

The electric double-layer capacitor of the present invention can be used for adsorption and preservation of an energy source, owing to excellent electrostatic capacity per unit volume. Particularly, it can be used for preservation of an energy source in the portable electronic terminal field and the transportation appliance field having a charging function, even in cold district, because of excellent electrostatic capacity under low temperature.

## Claims

1. An activated carbon obtained by activating a carbide of a compound represented by the formula (1) with an alkali metal hydroxide: 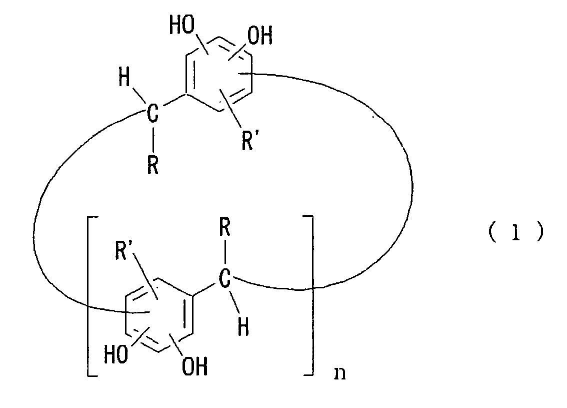 wherein, R represents a hydrocarbon group having 1 to 12 carbon atoms, and said hydrocarbon group may have a hydroxyl group, an alkyl group, an alkoxy group, an aryl group, an aryloxy group, a sulfonyl group, a halogen atom, a nitro group, a thioalkyl group, a cyano group, a carboxyl group, an amino group or an amide group, R' represents a hydrogen atom or a methyl group, and n represents 3, 5 or 7.

2. The activated carbon according to Claim 1, wherein R' in said formula (1) is a hydrogen atom.

3. The activated carbon according to Claim 1 or 2, wherein the alkali metal hydroxide is potassium hydroxide, sodium hydroxide or a mixture of potassium hydroxide and sodium hydroxide.

4. An electrode containing the activated carbon as described in any one of Claims 1 to 3.

5. An electric double-layer capacitor having the electrode as described in Claim 4.

6. A method for producing an activated carbon comprising carbonizing and activating a mixture of a compound represented by the formula (1) and an alkali metal hydroxide by heating under an inert gas atmosphere: 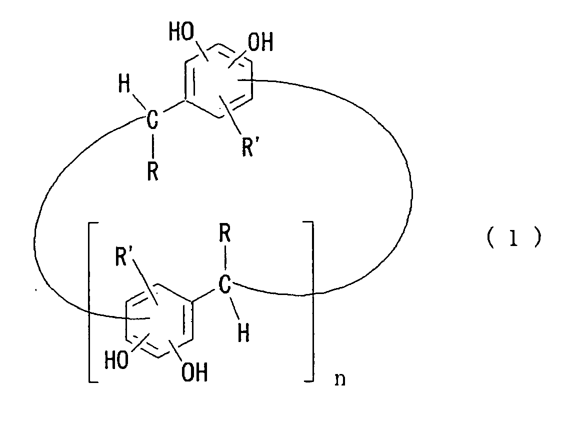 wherein, R represents a hydrocarbon group having 1 to 12 carbon atoms, and said hydrocarbon group may have a hydroxyl group, an alkyl group, an alkoxy group, an aryl group, an aryloxy group, a sulfonyl group, a halogen atom, a nitro group, a thioalkyl group, a cyano group, a carboxyl group, an amino group or an amide group, R' represents a hydrogen atom or a methyl group, and n represents 3, 5 or 7.

7. A method for producing an activated carbon comprising carbonizing a compound represented by the formula (1) under an inert gas atmosphere, then, mixing an alkali metal hydroxide, and heating under an inert gas atmosphere to cause activation: 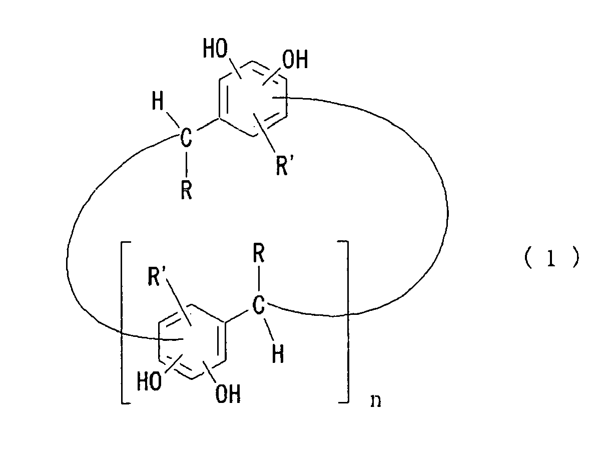 wherein, R represents a hydrocarbon group having 1 to 12 carbon atoms, and said hydrocarbon group may have a hydroxyl group, an alkyl group, an alkoxy group, an aryl group, an aryloxy group, a sulfonyl group, a halogen atom, a nitro group, a thioalkyl group, a cyano group, a carboxyl group, an amino group or an amide group, R' represents a hydrogen atom or a methyl group, and n represents 3, 5 or 7.
